# EUROPEAN PATENT APPLICATION

(11) **EP 4 567 116 A1**
(43) Date of publication of application: **11.06.2025**
(21) Application number: 23850081.3
(22) Date of filing: 01.08.2023
(51) Int. Cl.: C12N 15/31, C07K 14/195

(54) **OPTICAL SWITCH PROTEIN THAT FUNCTIONS THROUGH IRRADIATION WITH LONG WAVELENGTH LIGHT**

(30) Priority: 02.08.2022 JP 2022123061
(71) Applicant: The University of Tokyo, Bunkyo-ku, Tokyo 113-8654 (JP); KANAGAWA INSTITUTE OF INDUSTRIAL SCIENCE AND TECHNOLOGY, Ebina-shi Kanagawa 243-0435 (JP)
(72) Inventor: SATO Moritoshi, Tokyo 113-8654 (JP); NAKAJIMA Takahiro, Ebina-shi, Kanagawa 243-0435 (JP)
(74) Representative: Vossius & Partner Patentanwälte Rechtsanwälte mbB
(86) International application number: PCT/JP2023/028106
(87) International publication number: WO 2024/029527

(57) **Abstract**

It is an object of the present invention to provide an RpBphP1 mutant and an RpPpsR2 mutant that have been improved so that their binding and dissociation can be reliably controlled by light irradiation with a near-infrared light and blocking thereof. More specifically, the present invention relates to a protein set consisting of two proteins that bind to each other under near-infrared light irradiation, the protein set being a set of an RpBphP1 mutant and a wild-type RpPpsR2, a set of a wild-type RpBphP1 and an RpPpsR2 mutant, or a set of an RpBphP1 mutant and an RpPpsR2 mutant, wherein the binding of the protein set is weaker under the dark and/or is stronger under near-infrared light irradiation, than the binding of a wild-type RpBphP1 and a wild-type RpPpsR2.

## Description

### Technical Field

The present invention relates to a set of proteins whose binding and dissociation can be controlled by irradiation with a light.

### Background Art

Protein, which is one of biological materials, plays a various functions in a living body. For example, proteins are involved in the control of systems that are fundamental to life, such as intracellular signal transduction and material transport, through interactions between the proteins. In recent years, with the development of optogenetics technology, techniques have been developed to control interactions between proteins in a living body by light manipulation, and for example, photoswitching proteins are currently attracting attention.

Photoswitching proteins are a pair of proteins whose binding and dissociation can be controlled by turning light irradiation ON and OFF (Patent Literature 1). Through the binding and dissociation of such photoswitching proteins by light manipulation, various intracellular phenomena, such as signal transduction, genome editing and gene expression, can be controlled (Non Patent Literature 1 and Non Patent Literature 2). By the way, until now, photoswitching proteins that can be controlled with a blue light (up to 470 nm) have been widely used for light manipulation. However, since such a blue light is absorbed by hemoglobin, etc., and it has low permeability into biological tissues, photoswitching proteins that can be manipulated with a blue light have not exhibited sufficient effects for the use of light manipulation in vivo. Thus, it has been desired to use photoswitching proteins, which can be manipulated with a near-infrared light that is not absorbed by hemoglobin and has high permeability into biological tissues. At that time, a certain research group has reported that a photoreceptor (RpBphP1) possessed by bacteria (Rhodopseudomonas palustris) and a binding protein thereof (RpPpsR2) can be utilized as photoswitching proteins by irradiation with a near-infrared light (Non Patent Literatures 3 and 4).

The binding and dissociation of RpBphP1 and RpPpsR2 can be controlled by a near-infrared light, which has high permeability into biological tissues, and also, Biliverdin, a cofactor required for RpBphP1 to receive a light, is widely present in the cells of various animal species, including mammals. Therefore, it was expected that the pair of RpBphP1 and RpPpsR2 would be utilized in the medical field. However, since a pair of wild-type RpBphP1 and RpPpsR2 binds to each other even under the dark, namely, in the absence of light irradiation, this pair has been problematic in that it is difficult to reliably control the binding and dissociation of the pair by light irradiation.

### Citation List

### Patent Literature

Patent Literature 1: JP Patent Publication (Kokai) No. 2015-165776 A

### Non Patent Literature

Non Patent Literature 1: Kawano et al., Nature Chemical Biology 12: 1059-1064, 2016.
Non Patent Literature 2: Kawano et al., Nature Communications 6: 6256, 2015.
Non Patent Literature 3: Kaberniuk et al., Nature Methods 13: 591-597, 2016.
Non Patent Literature 4: Redchuk et al., Nature Chemical Biology 13: 633-639, 2017.

### Summary of Invention

### Technical Problem

In view of the above-described circumstances, it is an object of the present invention to provide an RpBphP1 mutant and an RpPpsR2 mutant that have been improved so that their binding and dissociation can be reliably controlled by light irradiation with a near-infrared light and blocking thereof.

### Solution to Problem

The present inventors have prepared various mutants of RpBphP1 and RpPpsR2. As a result, the present inventors have succeeded in finding a combination of a mutant and a wild type, or a combination of mutants, whose binding and dissociation can be reliably controlled by turning light irradiation with a near-infrared light ON/OFF.

Specifically, the present invention includes the following (1) to (13).
(1) A protein set consisting of two proteins that bind to each other under near-infrared light irradiation, the protein set being a set of an RpBphP1 mutant and a wild-type RpPpsR2, a set of a wild-type RpBphP1 and an RpPpsR2 mutant, or a set of an RpBphP1 mutant and an RpPpsR2 mutant, wherein the binding of the protein set is weaker under the dark and/or is stronger under near-infrared light irradiation, than the binding of a wild-type RpBphP1 and a wild-type RpPpsR2.
(2) The protein set according to the above (1), wherein the wild-type RpBphP1 is a protein consisting of the amino acid sequence as set forth in SEQ ID No: 1 or SEQ ID No: 2, or a protein having a sequence identity of 90% or more to the amino acid sequence as set forth in SEQ ID No: 1 or SEQ ID No: 2, and the wild-type RpPpsR2 is a protein consisting of the amino acid sequence as set forth in SEQ ID No: 3, or a protein having a sequence identity of 90% or more to the amino acid sequence as set forth in SEQ ID No: 3.
(3) The protein set according to the above (2), which is a set of a wild-type RpPpsR2 and an RpBphP1 mutant, wherein
   the RpBphP1 mutant is any of mutants consisting of amino acid sequences having one or multiple point mutations selected from the group of point mutations consisting of Q514A, D607E, R638A, H661A, R696A, E327A, E332A, E337A, D339A, E365A, Q367A, E429A, R490A, D498A, Q502A, Q502G, Q502V, Q502L, Q502I, Q502S, Q502T, Q502E, Q502K, Q502R, Q502H, Q502N, Q502F, Q502Y, Q502W, Q502C, Q502M, Q502P, F503A, R504A, R507A, E513G, E513D, E513P, E516A, F518A, S525A, D607A, D607G, D607V, D607L, D607I, D607S, D607T, D607K, D607R, D607H, D607N, D607Q, D607F, D607W, D607C, D607M, D607P, E613A, R636A, F639A, D654A, E678A, Y681A, E693A, H712D, H712E, H712P, R512A, E513V, E513A, E513L, E513T, E513M, and Q521A, in the amino acid sequence as set forth in SEQ ID No: 1 or SEQ ID No: 2.
(4) The protein set according to the above (2), which is a set of a wild-type RpBphP1 and an RpPpsR2 mutant, wherein
   the RpPpsR2 mutant is any of mutants consisting of amino acid sequences having one or multiple point mutations selected from the group of point mutations consisting of Q105T, T133A, L137A, N103A, Q105A, Q105C, Q105H, V107A, T108A, L110A, R113A, L114A, I115A, Q119A, M121A, M121E, R123A, D124A, Y125A, Y125E, W126A, R127A, R129A, E132A, R134A, R136A, Q105V, Q105S, K102A, Q105P, Q105R, L137E, V138A, V138E, F139E, and V146A, in the amino acid sequence as set forth in SEQ ID No: 3.
(5) The protein set according to the above (2), which is a set of a wild-type RpBphP1 and an RpPpsR2 mutant, wherein
   the RpPpsR2 mutant is a mutant consisting of an amino acid sequence, in which the amino acid sequence as set forth in SEQ ID No: 3 is modified to any of D1-D1-D2, D3-D1-D2, D1-D2 (del N 77), D1-D2, D2-D1, D2-D2, D3-D2, D3-D2 (L137E), D4-D2, D1-D2-D1, D1-D2-D2, D1-D2-D4, D2-D1-D2, D2-D3-D4, D4-D1-D2, D2-D3-D4-D1, D2-D3-D4-D2, D2-D3-D4-D3, D2-D3-D4-D5, D1-D2 (del N 88), D1-D2 (del N 89), D1-D2 (del N 90), D1-D2 (del N 91), D1-D2 (del N 92), D1-D2 (del N 93), D1-D2 (del N 94), D1-D2 (del N 95), D1-D2 (del N 96), D1-D2 (del N 97), D1-D2 (del C 5), D1-D2 (del C 15), D1-D2 (del N 93) (del C 5), D1-D2 (del N 93) (del C 6), D1-D2 (del N 93) (del C 11), D1-D2 (del N 93) (del C 12), D1-D2-D3 (del C 25), D1-D2-D3 (del C 41), D1-D2-D3 (del C 49), D1-D2-D3 (del C 70), D1-D2-D3 (del C 87), D1-D2-D3 (del C 96), D1-D2-D3 (del C 104), D1-D2 (del N 64), D2-D3, D1-D2-D3, D2-D2-D3, D2-D3-D1, D2-D3-D3, D3-D2-D3, D4-D2-D3, D1-D1-D2-D3, D1-D2-D3-D1, D1-D2-D3-D2, D1-D2-D3-D3, D1-D2-D3-D4, D2-D1-D2-D3, D2-D2-D3-D4, D2-D3-D4-D4, D3-D1-D2-D3, D3-D2-D3-D4, D1-D2 (del N 15), D1-D2 (del N 33), D1-D2 (del N 47), and D1-D2-D3 (del C 9).
(6) The protein set according to the above (2), which is a set of an RpBphP1 mutant and an RpPpsR2 mutant, wherein
   the RpBphP1 mutant is any of mutants consisting of amino acid sequences having one or multiple point mutations selected from the group of point mutations consisting of Q514A, D607E, R638A, H661A, R696A, E327A, E332A, E337A, D339A, E365A, Q367A, E429A, R490A, D498A, Q502A, Q502G, Q502V, Q502L, Q502I, Q502S, Q502T, Q502E, Q502K, Q502R, Q502H, Q502N, Q502F, Q502Y, Q502W, Q502C, Q502M, Q502P, F503A, R504A, R507A, E513G, E513D, E513P, E516A, F518A, S525A, D607A, D607G, D607V, D607L, D607I, D607S, D607T, D607K, D607R, D607H, D607N, D607Q, D607F, D607W, D607C, D607M, D607P, E613A, R636A, F639A, D654A, E678A, Y681A, E693A, H712D, H712E, H712P, R512A, E513V, E513A, E513L, E513T, E513M, and Q521A, in the amino acid sequence as set forth in SEQ ID No: 1 or SEQ ID No: 2, and
   the RpPpsR2 mutant is any of mutants consisting of amino acid sequences having one or multiple point mutations selected from the group of point mutations consisting of Q105T, T133A, L137A, N103A, Q105A, Q105C, Q105H, V107A, T108A, L110A, R113A, L114A, I115A, Q119A, M121A, M121E, R123A, D124A, Y125A, Y125E, W126A, R127A, R129A, E132A, R134A, R136A, Q105V, Q105S, K102A, Q105P, Q105R, L137E, V138A, V138E, F139E, and V146A, in the amino acid sequence as set forth in SEQ ID No: 3.
(7) The protein set according to the above (2), which is a set of an RpBphP1 mutant and an RpPpsR2 mutant, wherein
   the RpBphP1 mutant is any of mutants consisting of amino acid sequences having one or multiple point mutations selected from the group of point mutations consisting of Q514A, D607E, R638A, H661A, R696A, E327A, E332A, E337A, D339A, E365A, Q367A, E429A, R490A, D498A, Q502A, Q502G, Q502V, Q502L, Q502I, Q502S, Q502T, Q502E, Q502K, Q502R, Q502H, Q502N, Q502F, Q502Y, Q502W, Q502C, Q502M, Q502P, F503A, R504A, R507A, E513G, E513D, E513P, E516A, F518A, S525A, D607A, D607G, D607V, D607L, D607I, D607S, D607T, D607K, D607R, D607H, D607N, D607Q, D607F, D607W, D607C, D607M, D607P, E613A, R636A, F639A, D654A, E678A, Y681A, E693A, H712D, H712E, H712P, R512A, E513V, E513A, E513L, E513T, E513M, and Q521A, in the amino acid sequence as set forth in SEQ ID No: 1 or SEQ ID No: 2, and
   the RpPpsR2 mutant is a mutant consisting of an amino acid sequence, in which the amino acid sequence as set forth in SEQ ID No: 3 is modified to any of D1-D1-D2, D3-D1-D2, D1-D2 (del N 77), D1-D2, D2-D1, D2-D2, D3-D2, D3-D2 (L137E), D4-D2, D1-D2-D1, D1-D2-D2, D1-D2-D4, D2-D1-D2, D2-D3-D4, D4-D1-D2, D2-D3-D4-D1, D2-D3-D4-D2, D2-D3-D4-D3, D2-D3-D4-D5, D1-D2 (del N 88), D1-D2 (del N 89), D1-D2 (del N 90), D1-D2 (del N 91), D1-D2 (del N 92), D1-D2 (del N 93), D1-D2 (del N 94), D1-D2 (del N 95), D1-D2 (del N 96), D1-D2 (del N 97), D1-D2 (del C 5), D1-D2 (del C 15), D1-D2 (del N 93) (del C 5), D1-D2 (del N 93) (del C 6), D1-D2 (del N 93) (del C 11), D1-D2 (del N 93) (del C 12), D1-D2-D3 (del C 25), D1-D2-D3 (del C 41), D1-D2-D3 (del C 49), D1-D2-D3 (del C 70), D1-D2-D3 (del C 87), D1-D2-D3 (del C 96), D1-D2-D3 (del C 104), D1-D2 (del N 64), D2-D3, D1-D2-D3, D2-D2-D3, D2-D3-D1, D2-D3-D3, D3-D2-D3, D4-D2-D3, D1-D1-D2-D3, D1-D2-D3-D1, D1-D2-D3-D2, D1-D2-D3-D3, D1-D2-D3-D4, D2-D1-D2-D3, D2-D2-D3-D4, D2-D3-D4-D4, D3-D1-D2-D3, D3-D2-D3-D4, D1-D2 (del N 15), D1-D2 (del N 33), D1-D2 (del N 47), and D1-D2-D3 (del C 9).
(8) An RpBphP1 mutant consisting of an amino acid sequence having any one or multiple point mutations selected from the following point mutations in the amino acid sequence as set forth in SEQ ID No: 1 or SEQ ID No: 2: Q514A, D607E, R638A, H661A, R696A, E327A, E332A, E337A, D339A, E365A, Q367A, E429A, R490A, D498A, Q502A, Q502G, Q502V, Q502L, Q502I, Q502S, Q502T, Q502E, Q502K, Q502R, Q502H, Q502N, Q502F, Q502Y, Q502W, Q502C, Q502M, Q502P, F503A, R504A, R507A, E513G, E513D, E513P, E516A, F518A, S525A, D607A, D607G, D607V, D607L, D607I, D607S, D607T, D607K, D607R, D607H, D607N, D607Q, D607F, D607W, D607C, D607M, D607P, E613A, R636A, F639A, D654A, E678A, Y681A, E693A, H712D, H712E, H712P, R512A, E513V, E513A, E513L, E513T, E513M, and Q521A.
(9) An RpPpsR2 mutant consisting of an amino acid sequence having any one or multiple point mutations selected from the following point mutations in the amino acid sequence as set forth in SEQ ID No: 3:
   Q105T, T133A, L137A, N103A, Q105A, Q105C, Q105H, V107A, T108A, L110A, R113A, L114A, I115A, Q119A, M121A, M121E, R123A, D124A, Y125A, Y125E, W126A, R127A, R129A, E132A, R134A, R136A, Q105V, Q105S, K102A, Q105P, Q105R, L137E, V138A, V138E, F139E, and V146A.
(10) An RpPpsR2 mutant consisting of an amino acid sequence, in which the amino acid sequence as set forth in SEQ ID No: 3 is modified to any of the following:
   D1-D1-D2, D3-D1-D2, D1-D2 (del N 77), D1-D2, D2-D1, D2-D2, D3-D2, D3-D2 (L137E), D4-D2, D1-D2-D1, D1-D2-D2, D1-D2-D4, D2-D1-D2, D2-D3-D4, D4-D1-D2, D2-D3-D4-D1, D2-D3-D4-D2, D2-D3-D4-D3, D2-D3-D4-D5, D1-D2 (del N 88), D1-D2 (del N 89), D1-D2 (del N 90), D1-D2 (del N 91), D1-D2 (del N 92), D1-D2 (del N 93), D1-D2 (del N 94), D1-D2 (del N 95), D1-D2 (del N 96), D1-D2 (del N 97), D1-D2 (del C 5), D1-D2 (del C 15), D1-D2 (del N 93) (del C 5), D1-D2 (del N 93) (del C 6), D1-D2 (del N 93) (del C 11), D1-D2 (del N 93) (del C 12), D1-D2-D3 (del C 25), D1-D2-D3 (del C 41), D1-D2-D3 (del C 49), D1-D2-D3 (del C 70), D1-D2-D3 (del C 87), D1-D2-D3 (del C 96), D1-D2-D3 (del C 104), D1-D2 (del N 64), D2-D3, D1-D2-D3, D2-D2-D3, D2-D3-D1, D2-D3-D3, D3-D2-D3, D4-D2-D3, D1-D1-D2-D3, D1-D2-D3-D1, D1-D2-D3-D2, D1-D2-D3-D3, D1-D2-D3-D4, D2-D1-D2-D3, D2-D2-D3-D4, D2-D3-D4-D4, D3-D1-D2-D3, D3-D2-D3-D4, D1-D2 (del N 15), D1-D2 (del N 33), D1-D2 (del N 47), and D1-D2-D3 (del C 9).
(11) A nucleic acid encoding any of the RpBphP1 mutants according to the above (8).
(12) A nucleic acid encoding any of the RpPpsR2 mutants according to the above (9).
(13) A nucleic acid encoding any of the RpPpsR2 mutants according to the above (10).

It is to be noted that the preposition "to" used in the present description indicates a numerical value range including the numerical values located left and right of the preposition.

### Advantageous Effects of Invention

The photoswitching proteins according to the present invention (i.e., a combination (set) of an RpBphP1 mutant and a wild-type RpPpsR2, a combination (set) of a wild-type RpBphP1 and an RpPpsR2 mutant, or a combination (set) of an RpBphP1 mutant and an RpPpsR2 mutant) can reliably control their binding and dissociation by irradiation with a near-infrared light and blocking thereof. Therefore, the photoswitching proteins according to the present invention can reliably control their binding and dissociation even in a living body by a near-infrared light.

### Brief Description of Drawings

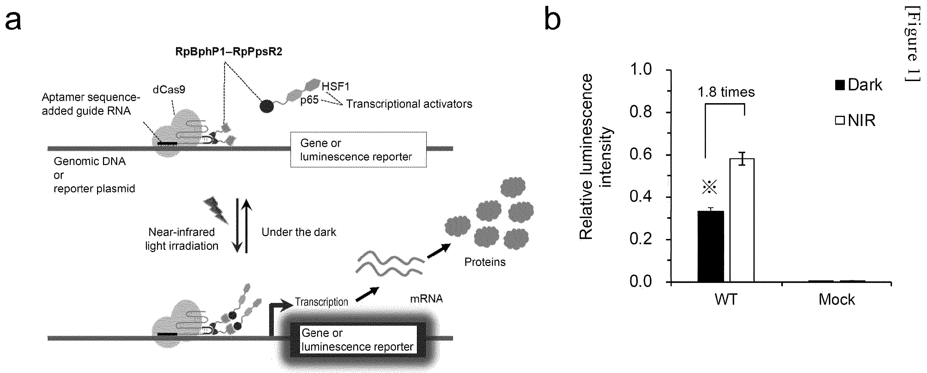

[Figure 1] Figure 1 shows confirmation of the functions of photoswitching proteins consisting of a wild-type RpBphP1 and a wild-type RpPpsR2. Figure 1(a) is a schematic view of CPTS (CRISPR-Cas9-based photoactivatable transcription system). Figure 1(b) shows the results obtained by confirming the binding state of a wild-type RpBphP1 and a wild-type RpPpsR2 under the dark and under near-infrared light irradiation.

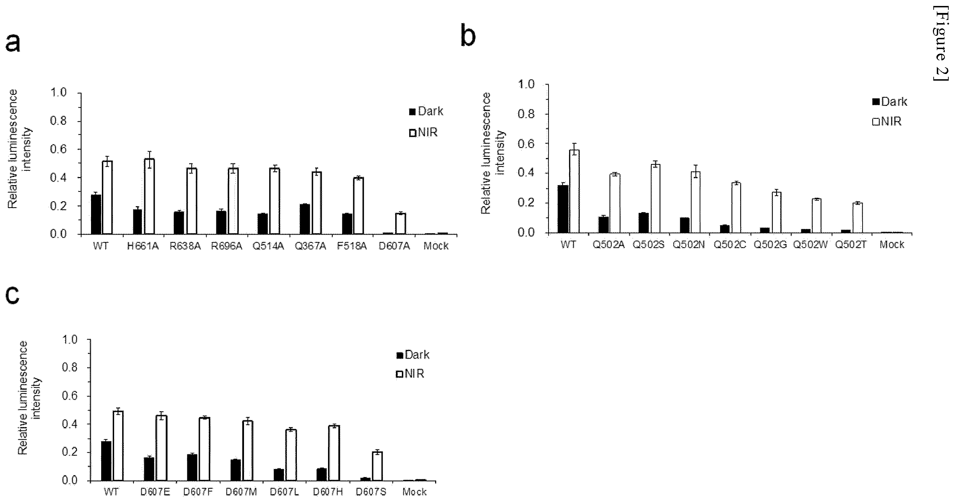

[Figure 2] Figure 2 shows the functional analysis of photoswitching proteins consisting of an RpBphP1 mutant and a wild-type RpPpsR2 (part 1). The results obtained by analyzing the binding state under the dark and under near-infrared light irradiation of an RpBphP1 with point mutations introduced and a wild-type RpPpsR2 are shown (Figures 2a to c).

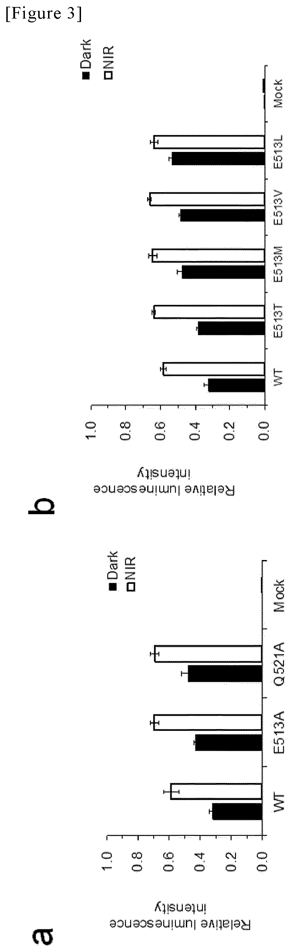

[Figure 3] Figure 3 shows the functional analysis of photoswitching proteins consisting of an RpBphP1 mutant and a wild-type RpPpsR2 (part 2). The results obtained by analyzing the binding state under the dark and under near-infrared light irradiation of an RpBphP1 with point mutations introduced and a wild-type RpPpsR2 are shown (Figures 3a and b).

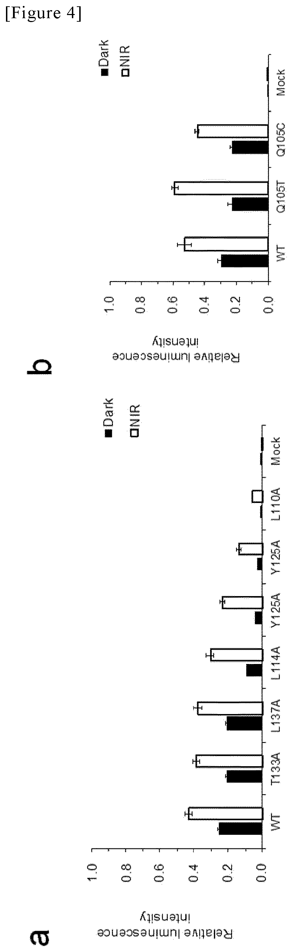

[Figure 4] Figure 4 shows the functional analysis of photoswitching proteins consisting of a wild-type RpBphP1 and an RpPpsR2 mutant (part 1). The results obtained by analyzing the binding state under the dark and under near-infrared light irradiation of an RpPpsR2 with point mutations introduced and a wild-type RpBphP1 are shown (Figures 4a and b).

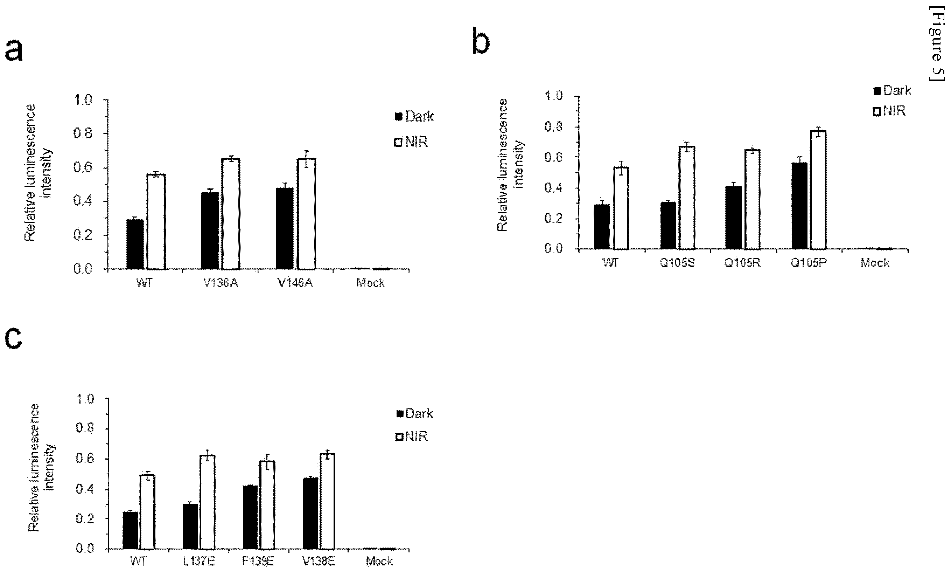

[Figure 5] Figure 5 shows the functional analysis of photoswitching proteins consisting of a wild-type RpBphP1 and an RpPpsR2 mutant (part 2). The results obtained by analyzing the binding state under the dark and under near-infrared light irradiation of an RpPpsR2 with point mutations introduced and a wild-type RpBphP1 are shown (Figures 5a to c).

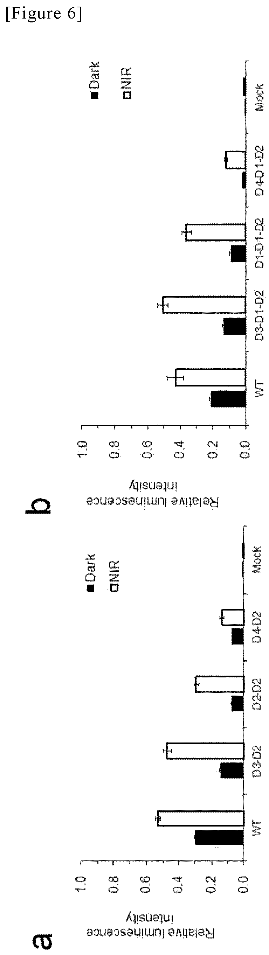

[Figure 6] Figure 6 shows the functional analysis of photoswitching proteins consisting of a wild-type RpBphP1 and an RpPpsR2 domain-modified body (part 1). The results obtained by analyzing the binding state under the dark and under near-infrared light irradiation of a wild-type RpBphP1 and an RpPpsR2 domain-modified body are shown (Figures 6a and b).

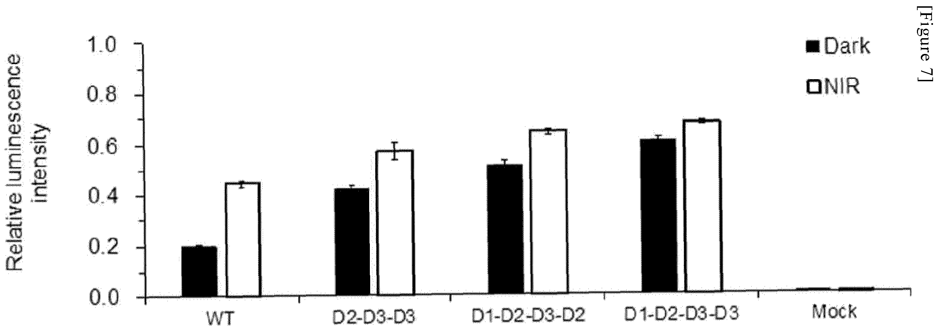

[Figure 7] Figure 7 shows the functional analysis of photoswitching proteins consisting of a wild-type RpBphP1 and an RpPpsR2 domain-modified body (part 2). The results obtained by analyzing the binding state under the dark and under near-infrared light irradiation of a wild-type RpBphP1 and an RpPpsR2 domain-modified body are shown.

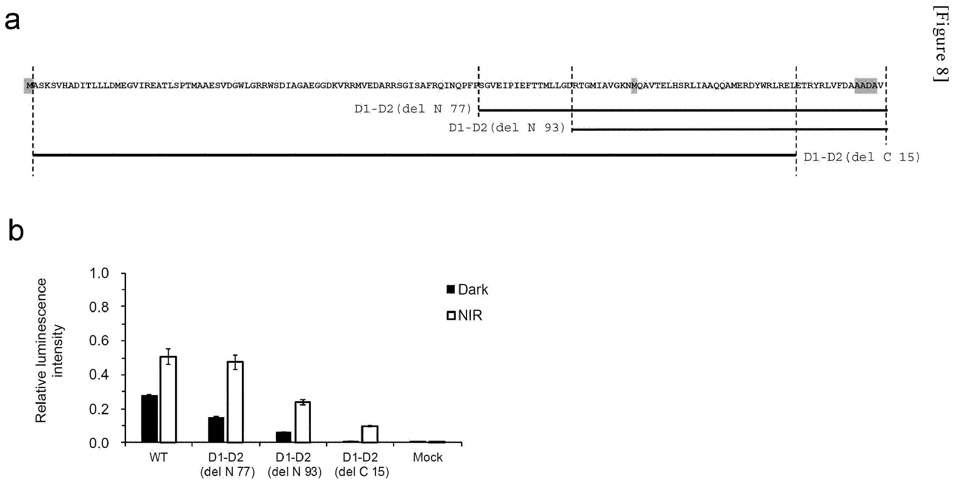

[Figure 8] Figure 8 shows the functional analysis of photoswitching proteins consisting of a wild-type RpBphP1 and an RpPpsR2 domain-modified body comprising a deletion in the domain thereof (part 1). The amino acid sequence (SEQ ID No: 6) shown in Figure 8a is the amino acid sequence of the D1-D2 domain of RpPpsR2. D1-D2 (del N 77), D1-D2 (del N 93), and D1-D2 (del C 15) indicate the amino acid sequences of the D1-D2 modified bodies having a deletion of 77 amino acids on the N-terminal side, a deletion of 93 amino acids on the N-terminal side, and a deletion of 15 amino acids on the C-terminal side, respectively. Figure 8b shows the results obtained by analyzing the binding state of a wild-type RpBphP1 and an RpPpsR2 domain-modified body comprising a deletion in the domain thereof under the dark and under near-infrared light irradiation.

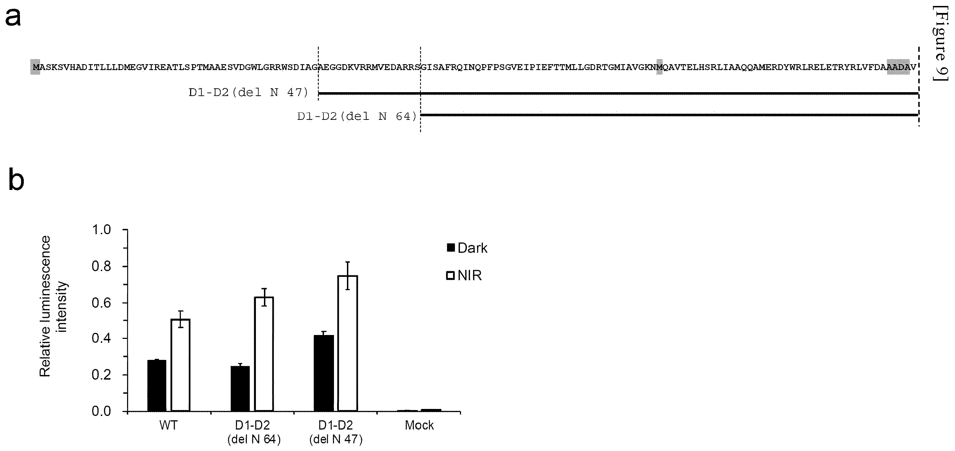

[Figure 9] Figure 9 shows the functional analysis of photoswitching proteins consisting of a wild-type RpBphP1 and an RpPpsR2 domain-modified body comprising a deletion in the domain thereof (part 2). The amino acid sequence (SEQ ID No: 6) shown in Figure 9a is the amino acid sequence of the D1-D2 domain of RpPpsR2. D1-D2 (del N 47) and D1-D2 (del N 64) indicate the amino acid sequences of the D1-D2 modified bodies having a deletion of 47 amino acids on the N-terminal side and a deletion of 64 amino acids on the N-terminal side, respectively. Figure 9b shows the results obtained by analyzing the binding state of a wild-type RpBphP1 and an RpPpsR2 domain-modified body comprising a deletion in the domain thereof under the dark and under near-infrared light irradiation.

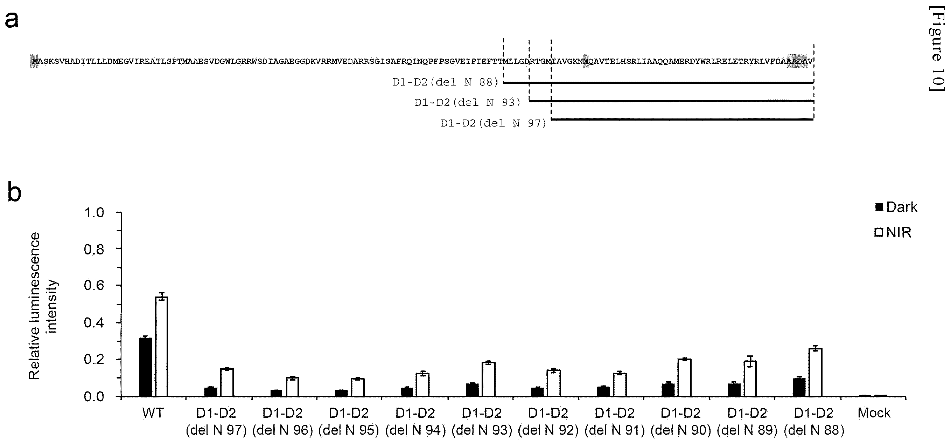

[Figure 10] Figure 10 shows the functional analysis of photoswitching proteins consisting of a wild-type RpBphP1 and an RpPpsR2 domain-modified body comprising a deletion in the domain thereof (part 3). The amino acid sequence (SEQ ID No: 6) shown in Figure 10a is the amino acid sequence of the D1-D2 domain of RpPpsR2. D1-D2 (del N 88), D1-D2 (del N 93), and D1-D2 (del N 97) indicate the amino acid sequences of the D1-D2 modified bodies having a deletion of 88 amino acids on the N-terminal side, a deletion of 93 amino acids on the N-terminal side, and a deletion of 97 amino acids on the N-terminal side, respectively. Figure 10b shows the results obtained by analyzing the binding state of a wild-type RpBphP1 and an RpPpsR2 domain-modified body comprising a deletion in the domain thereof under the dark and under near-infrared light irradiation.

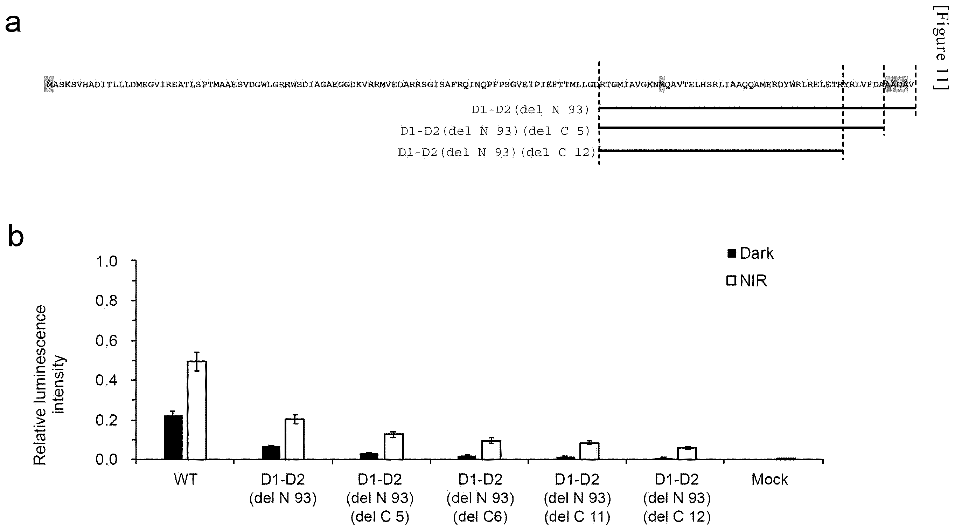

[Figure 11] Figure 11 shows the functional analysis of photoswitching proteins consisting of a wild-type RpBphP1 and an RpPpsR2 domain-modified body comprising a deletion in the domain thereof (part 4). The amino acid sequence (SEQ ID No: 6) shown in Figure 11a is the amino acid sequence of the D1-D2 domain of RpPpsR2. D1-D2 (del N 93), D1-D2 (del N 93) (del C 5), and D1-D2 (del N 93) (del C 12) indicate the amino acid sequences of the D1-D2 modified bodies having a deletion of 93 amino acids on the N-terminal side, a deletion of 93 amino acids on the N-terminal side and a deletion of 5 amino acids on the C-terminal side, and also, a deletion of 93 amino acids on the N-terminal side and a deletion of 12 amino acids on the C-terminal side, respectively. Figure 11b shows the results obtained by analyzing the binding state of a wild-type RpBphP1 and an RpPpsR2 domain-modified body comprising deletions in the domain thereof under the dark and under near-infrared light irradiation.

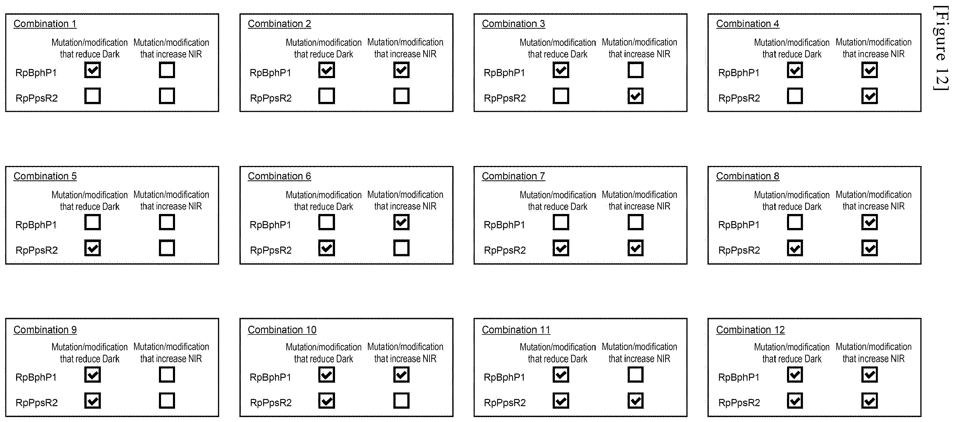

[Figure 12] Figure 12 shows combinations applied when a plurality of mutations and/or modifications having beneficial effects in RpBphP1 and RpPpsR2 (mutations and/or modifications that reduce Dark leak signals and mutations and/or modifications that increase NIR signals) are stacked together for function improvement. A total of 12 patterns are shown.

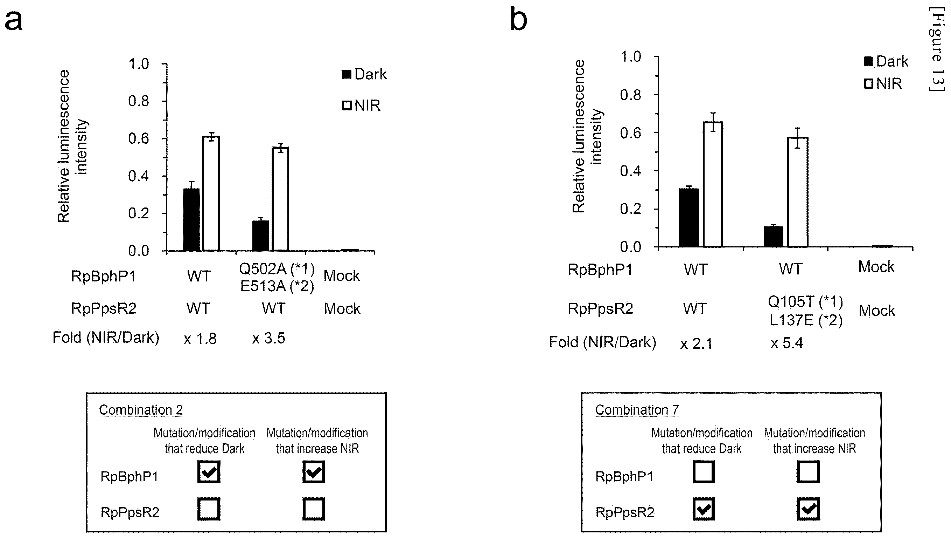

[Figure 13] Figure 13 shows the functional analysis of photoswitching proteins consisting of an RpBphP1 mutant having a plurality of beneficial point mutations and a wild-type RpPpsR2, and photoswitching proteins consisting of an RpPpsR2 mutant having a plurality of beneficial point mutations and a wild-type RpBphP1. Figure 13(a) shows an example in which the combination pattern of the RpBphP1 and the RpPpsR2 is Combination 2 shown in Figure 12. Figure 13(a) shows the results obtained by analyzing the binding state under the dark and under near-infrared light irradiation of an RpBphP1 mutant (with Q502A and E513A introduced) and a wild-type RpPpsR2. Figure 13(b) shows an example in which the combination pattern of the RpBphP1 and the RpPpsR2 is Combination 7 shown in Figure 12. Figure 13(b) shows the results obtained by analyzing the binding state under the dark and under near-infrared light irradiation of a wild-type RpBphP1 and an RpPpsR2 mutant (with Q105T and L137E introduced). *1 indicates a point mutation that reduces Dark leak signals. *2 indicates a point mutation that increases NIR signals.

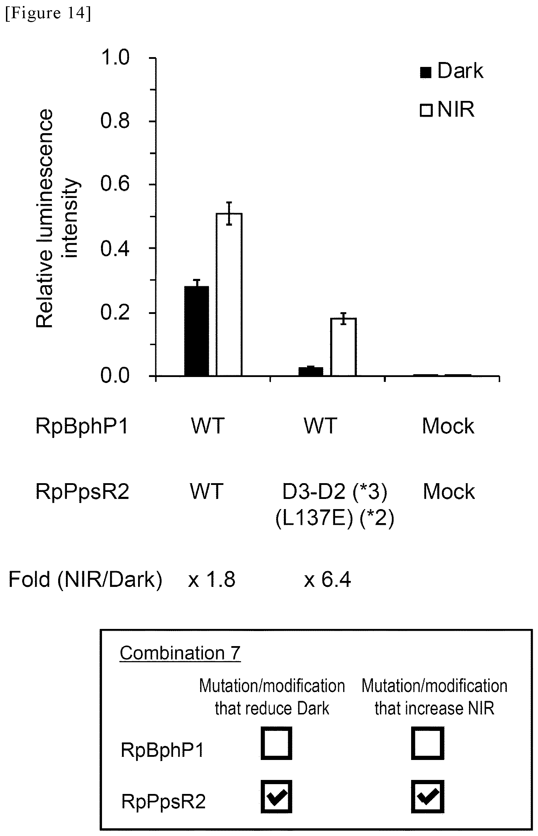

[Figure 14] Figure 14 shows the functional analysis of photoswitching proteins consisting of an RpPpsR2 mutant having both a beneficial domain modification and a point mutation, and a wild-type RpBphP1. This is an example in which the combination pattern of the RpBphP1 and the RpPpsR2 is Combination 7 shown in Figure 12. Here are shown the results obtained by analyzing the binding state under the dark and under near-infrared light irradiation of a wild-type RpBphP1 and an RpPpsR2 mutant having both a domain modification (D3-D2) and a point mutation (with L137E introduced). This combination pattern is the same as that in Figure 13b, with the exception that a domain modification and a point mutation have been introduced into the RpPpsR2. *2 indicates a point mutation that increases NIR signals. *3 indicates a domain-modified body that reduces Dark leak signals.

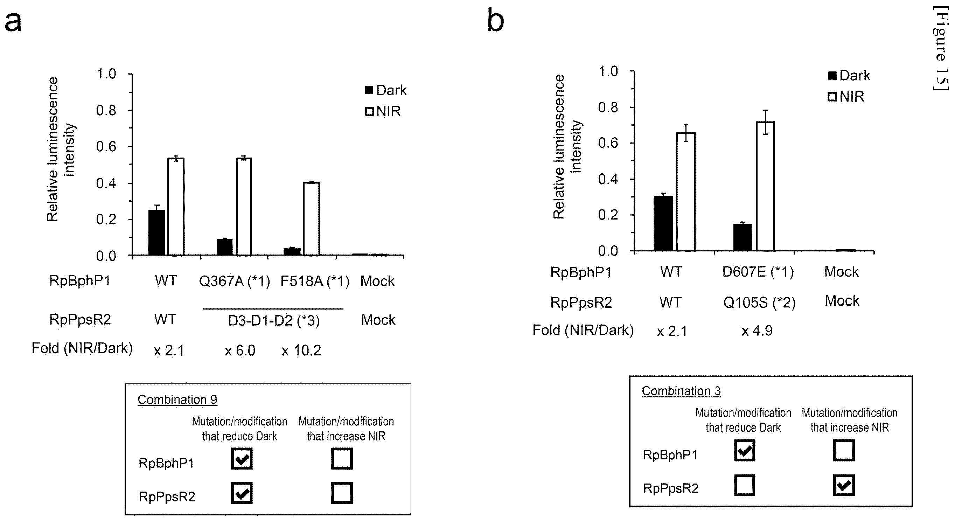

[Figure 15] Figure 15 shows the functional analysis of photoswitching proteins consisting of an RpBphP1 and an RpPpsR2, into both of which beneficial mutations have been introduced (Part 1). Figure 15(a) shows an example in which the combination pattern of the RpBphP1 and the RpPpsR2 is Combination 9 shown in Figure 12. Here are shown the results obtained by analyzing the binding state under the dark and under near-infrared light irradiation of an RpBphP1 mutant (with Q367A or F518A introduced) and an RpPpsR2 domain-modified body(D3-D1-D2). Figure 15(b) shows an example in which the combination pattern of the RpBphP1 and the RpPpsR2 is Combination 3 shown in Figure 12. Here are shown the results obtained by analyzing the binding state under the dark and under near-infrared light irradiation of an RpBphP1 mutant (with D607E introduced) and an RpPpsR2 mutant (with Q105S introduced). *1 indicates a point mutation that reduces Dark leak signals.. *2 indicates a point mutation that increases NIR signals. *3 indicates a domain-modified body that reduces Dark leak signals.

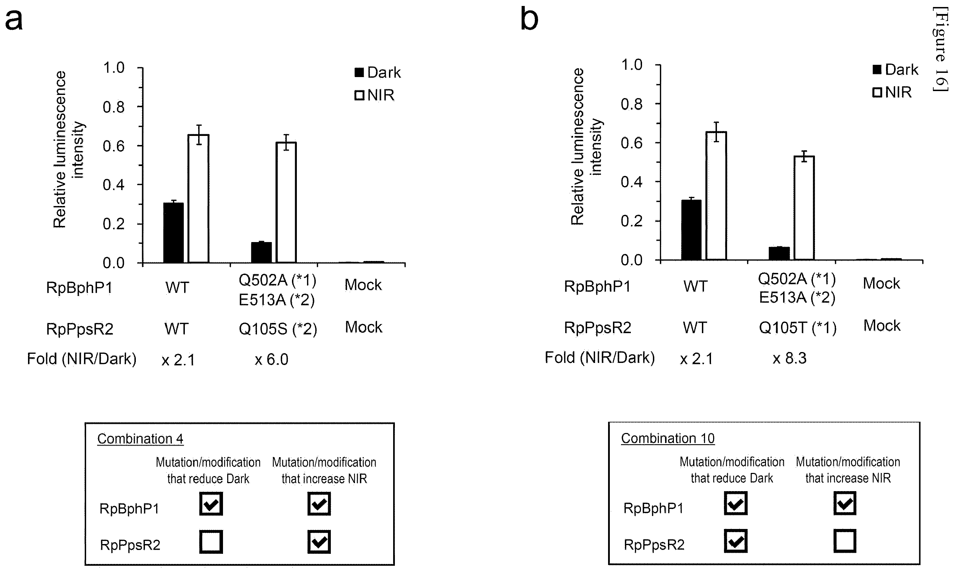

[Figure 16] Figure 16 shows the functional analysis of photoswitching proteins consisting of an RpBphP1 and an RpPpsR2, into both of which beneficial mutations have been introduced (part 2). Figure 16(a) shows an example in which the combination pattern of the RpBphP1 and the RpPpsR2 is Combination 4 shown in Figure 12. Here are shown the results obtained by analyzing the binding state under the dark and under near-infrared light irradiation of an RpBphP1 mutant (with Q502A and E513A introduced) and an RpPpsR2 mutant (with Q105S introduced). Figure 16(b) shows an example in which the combination pattern of the RpBphP1 and the RpPpsR2 is Combination 10 shown in Figure 12. Here are shown the results obtained by analyzing the binding state under the dark and under near-infrared light irradiation of an RpBphP1 mutant (with Q502A and E513A introduced) and an RpPpsR2 mutant (Q105T). *1 indicates a point mutation that reduces Dark leak signals.. *2 indicates a point mutation that increases NIR signals.

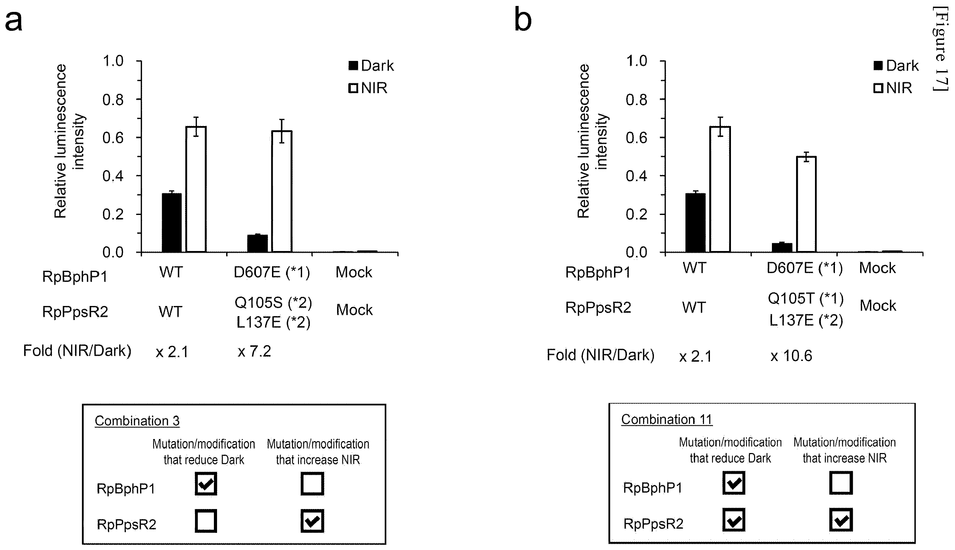

[Figure 17] Figure 17 shows the functional analysis of photoswitching proteins consisting of an RpBphP1 and an RpPpsR2, into both of which beneficial mutations have been introduced (part 3). Figure 17(a) shows an example in which the combination pattern of the RpBphP1 and the RpPpsR2 is Combination 3 shown in Figure 12. Here are shown the results obtained by analyzing the binding state under the dark and under near-infrared light irradiation of an RpBphP1 mutant (with D607E introduced) and an RpPpsR2 mutant (with Q105S and L137E introduced). This combination pattern is the same as that in Figure 15b, with the exception that two point mutations have been introduced into the RpPpsR2. Figure 17(b) shows an example in which the combination pattern of the RpBphP1 and the RpPpsR2 is Combination 11 shown in Figure 12. Here are shown the results obtained by analyzing the binding state under the dark and under near-infrared light irradiation of an RpBphP1 mutant (with D607E introduced) and an RpPpsR2 mutant(with Q105T and L137E introduced). *1 indicates a point mutation that reduces Dark leak signals.. *2 indicates a point mutation that increases NIR signals.

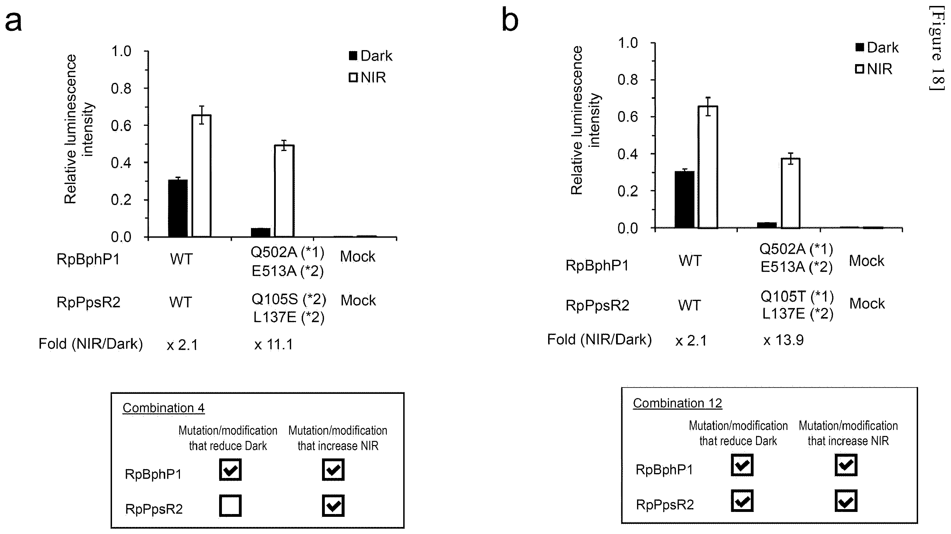

[Figure 18] Figure 18 shows the functional analysis of photoswitching proteins consisting of an RpBphP1 and an RpPpsR2, into both of which beneficial mutations have been introduced (part 4). Figure 18(a) shows an example in which the combination pattern of the RpBphP1 and the RpPpsR2 is Combination 4 shown in Figure 12. Here are shown the results obtained by analyzing the binding state under the dark and under near-infrared light irradiation of an RpBphP1 mutant (with Q502A and E513A introduced) and an RpPpsR2 mutant (with Q105S and L137E introduced). This combination pattern is the same as that in Figure 16a, with the exception that two point mutations have been introduced into the RpPpsR2. Figure 18(b) shows an example in which the combination pattern of the RpBphP1 and the RpPpsR2 is Combination 12 shown in Figure 12. Here are shown the results obtained by analyzing the binding state under the dark and under near-infrared light irradiation of an RpBphP1 mutant (with Q502A and E513A introduced) and an RpPpsR2 mutant (with Q105T and L137E introduced). *1 indicates a point mutation that reduces Dark leak signals.. *2 indicates a point mutation that increases NIR signals.

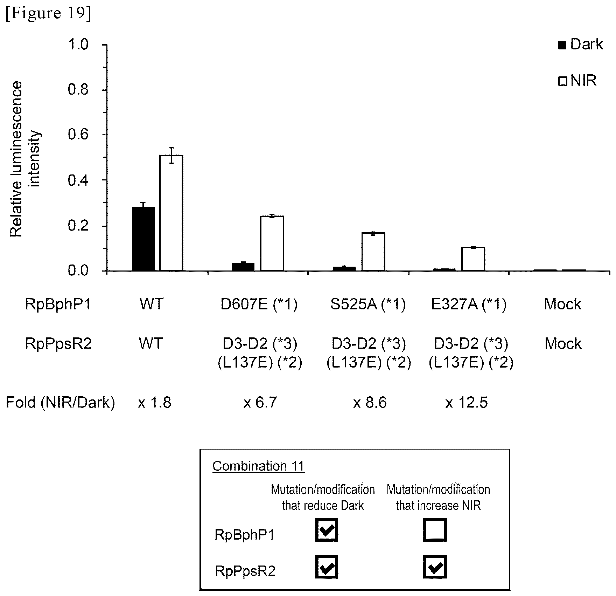

[Figure 19] Figure 19 shows the functional analysis of photoswitching proteins consisting of an RpBphP1 and an RpPpsR2, into both of which beneficial mutations have been introduced (part 5). This figure shows an example in which the combination pattern of the RpBphP1 and the RpPpsR2 is Combination 11 shown in Figure 12. Here are shown the results obtained by analyzing the binding state under the dark and under near-infrared light irradiation of an RpBphP1 mutant (with D609E, S525A or E327A introduced) and an RpPpsR2 mutant having both a domain modification (D3-D2) and a point mutation (with L137E introduced). This combination pattern is the same as that in Figure 17b, with the exception that a domain modification and a point mutation have been introduced into the RpPpsR2. *1 indicates a point mutation that reduces Dark leak signals.. *2 indicates a point mutation that increases NIR signals. *3 indicates a domain-modified body that reduces Dark leak signals.

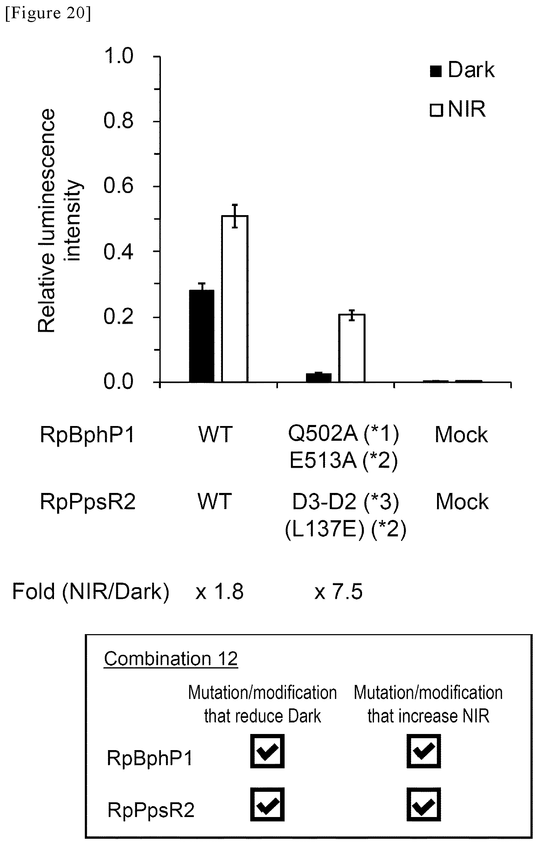

[Figure 20] Figure 20 shows the functional analysis of photoswitching proteins consisting of an RpBphP1 and an RpPpsR2, into both of which beneficial mutations have been introduced (part 6). This figure shows an example in which the combination pattern of the RpBphP1 and the RpPpsR2 is Combination 12 shown in Figure 12. Here are shown the results obtained by analyzing the binding state under the dark and under near-infrared light irradiation of an RpBphP1 mutant (with Q502A and E513A introduced) and an RpPpsR2 mutant having a domain modification (D3-D2) and a point mutation (with L137E introduced). This combination pattern is the same as that in Figure 18b, with the exception that a domain modification and a point mutation have been introduced into the RpPpsR2. *1 indicates a point mutation that reduces Dark leak signals.. *2 indicates a point mutation that increases NIR signals. *3 indicates a domain-modified body that reduces Dark leak signals.

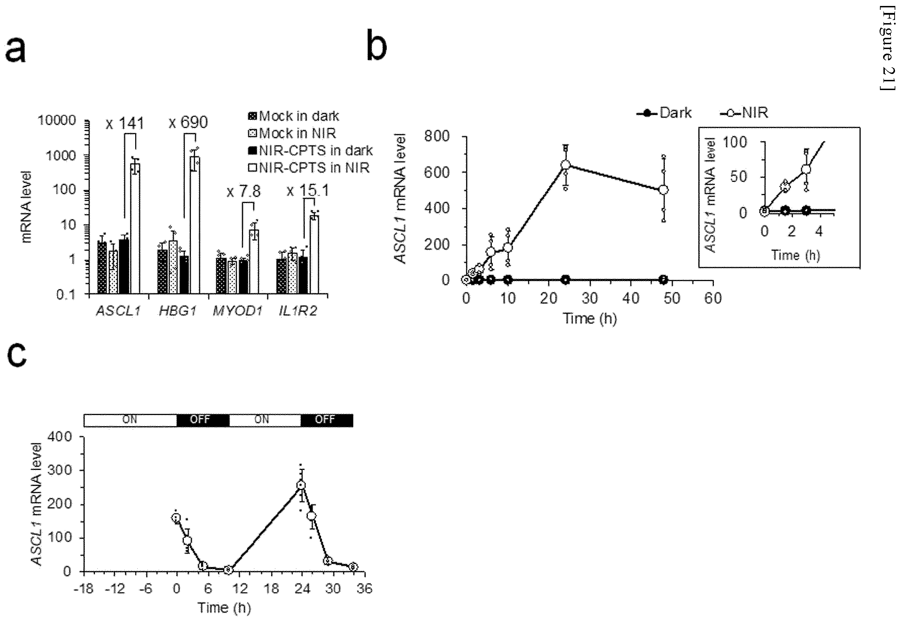

[Figure 21] Figure 21 shows confirmation of genome gene activation in cells by the photoswitching proteins of the present invention. Necessary components, such as guide RNA and a photoswitching protein gene targeting a genome gene (ASCL1, HBG1, MYOD1 or IL1R2), were transfected into HEK293T cells, and near-infrared light irradiation was then performed to examine the expression level of each genome gene. Figure 21a shows the expression level of each genome gene under the dark and under near-infrared light irradiation. Figure 21b shows a change over time in the expression level of ASCL1 mRNA after near-infrared light irradiation. The inside of the box shows an enlarged view up to 3 hours after the near-infrared light irradiation. Figure 21c shows the results obtained by examining how ASCL1 mRNA expression is influenced by ON or OFF of the near-infrared light irradiation.

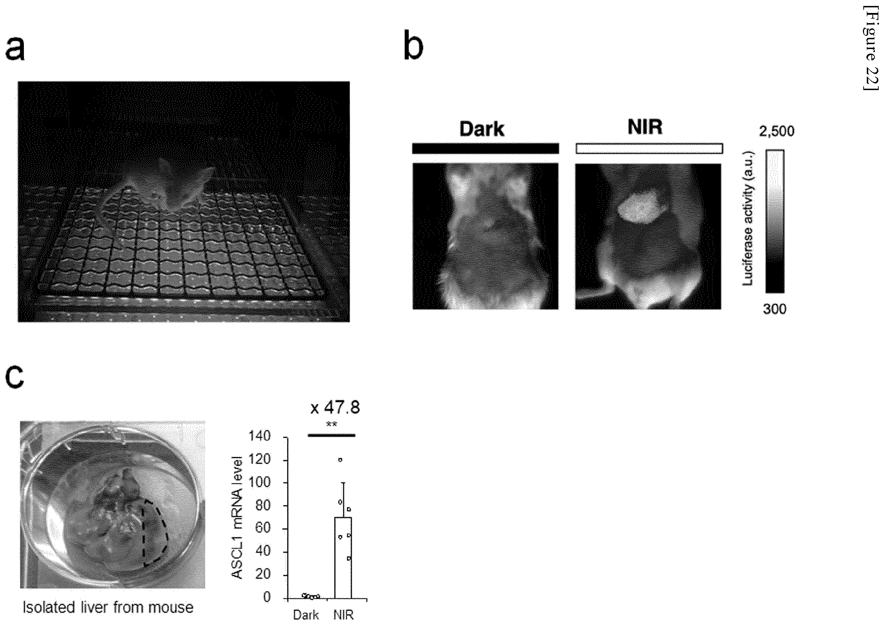

[Figure 22] Figure 22 shows confirmation of gene activation in the living bodies of mice by the photoswitching proteins of the present invention. Necessary components, such as guide RNA (targeting a luminescence reporter gene or targeting a mouse ASCL1 gene) and a photoswitching protein gene, were transfected into mouse liver, and the mice were then exposed to light irradiation (760 nm, 300 W/m²) using an LED panel from outside the mouse bodies. Figure 22a shows a scene of a near-infrared light irradiation experiment. Figure 22b shows the analysis results using a luminescence reporter gene. Figure 22c (left view) shows the liver excised from a mouse for the analysis of the expression level of an ASCL1 gene. Figure 22c (right view) shows the results obtained by examining the expression level of an ASCL1 gene in mouse liver under the dark and under near-infrared light irradiation.

### Description of Embodiments

Hereinafter, the embodiments of the present invention will be described.

A first embodiment relates to a protein set consisting of two proteins that bind to each other under near-infrared light irradiation (also referred to as "the protein set according to the present embodiment" or "the photoswitching proteins according to the present embodiment"),
the protein set being a set of an RpBphP1 mutant and a wild-type RpPpsR2, a set of a wild-type RpBphP1 and an RpPpsR2 mutant, or a set of an RpBphP1 mutant and an RpPpsR2 mutant, wherein
the binding of the protein set is weaker under the dark and/or is stronger under near-infrared light irradiation, than the binding of a wild-type RpBphP1 and a wild-type RpPpsR2.

As mentioned above, since the pair of a wild-type RpBphP1 and a wild-type RpPpsR2 binds to each other to some extent even under the dark, namely, in the absence of light irradiation, it has been difficult to reliably control the binding and dissociation of RpBphP1 and RpPpsR2 by turning light irradiation ON/OFF.

Thus, the present inventors have first attempted to create a protein pair with weaker binding under the dark and/or a protein pair with stronger binding under near-infrared light irradiation, compared to the binding between a wild-type RpBphP1 and a wild-type RpPpsR2. Specifically, individual mutants as described below were attempted to be created, namely, the binding of an RpBphP1 mutant and a wild-type RpPpsR2, the binding of a wild-type RpBphP1 and an RpPpsR2 mutant, and the binding of an RpBphP1 mutant and an RpPpsR2 mutant are:
1. weak in a state in which a near-infrared light is not irradiated (hereinafter also referred to as "under the dark"), and/or
2. strong under irradiation with a near-infrared light.

Herein, the "near-infrared light" refers to a light with a wavelength of approximately 650 nm to 900 nm.

Also, the phenomenon, in which the binding of the protein set according to the present embodiment is "weak" in comparison with the binding of a wild-type RpBphP1 and a wild-type RpPpsR2, means that the binding power evaluated by a reporter assay or the like described below is 0.6 times or less, preferably 0.4 times or less, and more preferably 0.2 times or less, of the binding power of a wild-type RpBphP1 and a wild-type RpPpsR2. Also, the phenomenon, in which the binding of the protein set according to the present embodiment is "strong" in comparison with the binding of a wild-type RpBphP1 and a wild-type RpPpsR2, means that the binding power is 1.1 times or more, and preferably 1.5 times or more, of the binding power of a wild-type RpBphP1 and a wild-type RpPpsR2.

As mentioned above, in the case of the pair of a wild-type RpBphP1 and a wild-type RpPpsR2, the binding power under near-infrared light irradiation is at most about twice that under the dark. With this degree of change in the binding power, it is difficult to precisely control the binding and dissociation of the pair by turning the near-infrared light irradiation ON/OFF. The protein set according to the present embodiment has a much larger difference in the binding power between under the dark and under near-infrared light irradiation, compared to a pair of wild types, and the binding and dissociation can be more precisely controlled. Specifically, with regard to the protein set according to the present embodiment, the binding power under near-infrared light irradiation is preferably about 3 times or more, more preferably about 5 times or more, and further preferably about 7 times or more than the binding power under the dark.

It is to be noted that, in the present description, the term "about" means a numerical value range of ±10%.

In the present embodiment, the wild-type RpBphP1 is a protein consisting of the amino acid sequence as set forth in SEQ ID No: 1 or SEQ ID No: 2 shown below, or a protein substantially identical to the protein consisting of the amino acid sequence as set forth in SEQ ID No: 1 or SEQ ID No: 2. Herein, the "protein substantially identical to the protein consisting of the amino acid sequence as set forth in SEQ ID No: 1 or SEQ ID No: 2" is a protein consisting of an amino acid sequence having a sequence identity of 80% or more to the amino acid sequence as set forth in SEQ ID No: 1 or SEQ ID No: 2, and is more preferably a protein consisting of an amino acid sequence having a sequence identity of 90% or more, 91% or more, 92% or more, 93% or more, 94% or more, 95% or more, 96% or more, 97% or more, 98% or more, or 99% or more, to the amino acid sequence as set forth in SEQ ID No: 1 or SEQ ID No: 2. However, the RpBphP1 mutant according to the present embodiment is excluded.

SEQ ID No: 1: 
MVAGHASGSPAFGTADLSNCEREEIHLAGSIQPHGALLVVSEPDHRIIQASA
NAAEFLNLGSVLGVPLAEIDGDLLIKILPHLDPTAEGMPVAVRCRIGNPSTE
YDGLMHRPPEGGLIIELERAGPPIDLSGTLAPALERIRTAGSLRALCDDTALL
FQQCTGYDRVMVYRFDEQGHGEVFSERHVPGLESYFGNRYPSSDIPQMAR
 
RLYERQRVRVLVDVSYQPVPLEPRLSPLTGRDLDMSGCFLRSMSPIHLQYL
KNMGVRATLVVSLVVGGKLWGLVACHHYLPRFIHFELRAICELLAEAIATRI
TALESFAQSQSELFVQRLEQRMIEAITREGDWRAAIFDTSQSILQPLHADGC
ALVYEDQIRTIGDVPSTQDVREIAGWLDRQPRAAVTSTASLGLDVPELAHL
TRMASGVVAAPISDHRGEFLMWFRPERVHTVTWGGDPKKPFTMGDTPAD
LSPRRSFAKWHQVVEGTSDPWTAADLAAARTIGQTVADIVLQFRAVRTLIA
REQYEQFSSQVHASMQPVLITDAEGRILLMNDSFRDMLPAGSPSAVHLDDL
AGFFVESNDFLRNVAELIDHGRGWRGEVLLRGAGNRPLPLAVRADPVTRT
EDQSLGFVLIFSDATDRRTADAARTRFLEGILASARPGVRLDSKSDLLHEKL
LSALVENAQLAALEITYGVETGRIAELLEGVRQSMLRTAEVLGHLVQHAA
RTAGSDSSSNGSQN
SEQ ID No: 2:

MAGHASGSPAFGTADLSNCEREEIHLAGSIQPHGALLVVSEPDHRIIQASAN
AAEFLNLGSVLGVPLAEIDGDLLIKILPHLDPTAEGMPVAVRCRIGNPSTEY
DGLMHRPPEGGLIIELERAGPPIDLSGTLAPALERIRTAGSLRALCDDTALLF
QQCTGYDRVMVYRFDEQGHGEVFSERHVPGLESYFGNRYPSSDIPQMARR
LYERQRVRVLVDVSYQPVPLEPRLSPLTGRDLDMSGCFLRSMSPIHLQYLK
NMGVRATLVVSLVVGGKLWGLVACHHYLPRFIHFELRAICELLAEAIATRIT
ALESFAQSQSELFVQRLEQRMIEAITREGDWRAAIFDTSQSILQPLHADGCA
LVYEDQIRTIGDVPSTQDVREIAGWLDRQPRAAVTSTASLGLDVPELAHLT
RMASGVVAAPISDHRGEFLMWFRPERVHTVTWGGDPKKPFTMGDTPADL
SPRRSFAKWHQVVEGTSDPWTAADLAAARTIGQTVADIVLQFRAVRTLIAR
EQYEQFSSQVHASMQPVLITDAEGRILLMNDSFRDMLPAGSPSAVHLDDLA
GFFVESNDFLRNVAELIDHGRGWRGEVLLRGAGNRPLPLAVRADPVTRTE
DQSLGFVLIFSDATDRRTADAARTRFQEGILASARPGVRLDSKSDLLHEKLL
SALVENAQLAALEITYGVETGRIAELLEGVRQSMLRTAEVLGHLVQHAART
AGSDSSSNGSQNKK

It is to be noted that the amino acid sequence of SEQ ID No: 2 is a sequence registered in the NCBI database under Accession No. WP_119846077. The amino acid sequence as set forth in SEQ ID No: 1 is the sequence used as the amino acid sequence of a wild-type RpBphP1 in the present Examples, in which V is inserted between the first M and the next A in the amino acid sequence as set forth in SEQ ID No: 2, and also, the 641st Q in the amino acid sequence as set forth in SEQ ID No: 2 is changed to L. These mutations have been made to, for example, improve the expression rate of the wild-type RpBphP1, and it has been confirmed that the amino acid sequence of SEQ ID No: 1 and the amino acid sequence of SEQ ID No: 2 have a sequence identity of 99% or more, and that the RpBphP1 mutant has the same activity as RpBphP1.

It is to be noted that, in the present description, the 3rd A in SEQ ID No: 1 (i.e., the 2nd A in SEQ ID No: 2) is used as amino acid No. 1 to indicate mutants of RpBphP1.

In the present embodiment, the wild-type RpPpsR2 is a protein consisting of the amino acid sequence as set forth in SEQ ID No: 3 shown below, or a protein substantially identical to the protein consisting of the amino acid sequence as set forth in SEQ ID No: 3. Herein, the "protein substantially identical to the protein consisting of the amino acid sequence as set forth in SEQ ID No: 3" is a protein consisting of an amino acid sequence having a sequence identity of 80% or more to the amino acid sequence as set forth in SEQ ID No: 3, and is more preferably a protein consisting of an amino acid sequence having a sequence identity of 90% or more, 91% or more, 92% or more, 93% or more, 94% or more, 95% or more, 96% or more, 97% or more, 98% or more, or 99% or more, to the amino acid sequence as set forth in SEQ ID No: 3. However, the RpPpsR2 mutant according to the present embodiment is excluded.

SEQ ID No: 3:
MASKSVHADITLLLDMEGVIREATLSPTMAAESVDGWLGRRWSDIAGAEG
GDKVRRMVEDARRSGISAFRQINQPFPSGVEIPIEFTTMLLGDRTGMIAVGK
NMQAVTELHSRLIAAQQAMERDYWRLRELETRYRLVFDAAADAVMIVSA
GDMRIVEANRAAVNAISRVERGNDDLAGRDFLAEVAAADRDAVRDMLAQ
VRQRGTALSVLVHLGRYDRAWMLRGSLMSSERRQVFLLHFTPVTTTPAID
DVDDDAVLRGLIDRIPDGFVALDSEGVVRHANQAFLDLVQIGSKPAAVGRS
LGVWMGRPGADLSSLLTLLRRYKTVRLFQTTIRGELGTETEVEVSAVDGE
DDQYIGVLMRNVARRLDAADDHDALRQALGPISKQLGRSSLRKLVKNAVS
IVEQHYVKEALLRSKGNRTATAELLGLSRQSLYAKLNSYGFDDKGVVASAA
DGAEGASDDAED

In the present embodiment, when a specific amino acid on an amino acid sequence is indicated, it is described in the order of the "specific amino acid (one-letter notation)" and "amino acid position number" in the amino acid sequence. For example, glutamine at position 514 on an amino acid sequence is indicated as "Q514." In addition, in the present embodiment, a point mutation is indicated in the order of an "amino acid," an "amino acid position number," and a "substituted amino acid" in a wild-type sequence. For example, a mutation in which the amino acid at position 514 in the wild-type RpBphP1 is substituted from Q to A is indicated as "Q514A," and a mutant having the present mutation is indicated as an "RpBphP1 mutant having a point mutation of Q514A," or the like. Similarly, a mutation in which the amino acid at position 105 in the wild-type RpPpsR2 is substituted from Q to T is indicated as "Q105T", and a mutant having the present mutation is indicated as an "RpPpsR2 mutant having a point mutation of Q105T," or the like. Moreover, in the case of a mutant having multiple point mutations, it is indicated, for example, as an "RpBphP1 mutant having point mutations of Q514A and D607E (i.e., an RpBphP1 mutant, in which Q at position 514 is substituted with A, and D at position 607 is substituted with E)," or the like.

Regarding the RpPpsR2 mutant, the present embodiment also includes mutants in which the positions of 5 domains constituting the wild-type RpPpsR2 are swapped, mutants in which one or more of the 5 domains are partially or completely deleted, and mutants in which the positions of the 5 domains are swapped and one or more of the 5 domains are partially or completely deleted, in addition to point mutants. These domain-related mutants are also referred to as "domain-modified bodies." In the present description, the positions of the 5 domains of the RpPpsR2 are defined as follows:
in the amino acid sequence as set forth in SEQ ID No: 3,
Domain 1 (also referred to as D1): from A2 to N103;
Domain 2 (also referred to as D2): from Q105 to A141;
Domain 3 (also referred to as D3): from V146 to T241;
Domain 4 (also referred to as D4): from D253 to N362; and
Domain 5 (also referred to as D5): From A369 to D464.

Herein, the amino acid sequences among the domains, for example, amino acid residues or amino acid sequences (also referred to as "linkers," etc.) among the domains, such as, for example, M104, A142 to A145, P242 to V252, and V363 to D368, are considered to be amino acids or amino acid linkers connecting the domains. However, in addition to the amino acid sequences of individual domains defined above, a number of amino acid residues (for example, about 1 to 10 residues) may be added to the N-terminus and/or C-terminus of the domains of the present embodiment, or may be deleted from the domains of the present embodiment, as long as the functions of individual domains are not impaired. Specifically, for example, in the case of a domain-modified body represented as D2-D1-D4, the polypeptide (D2) ranging from Q105 to A141 of the amino acid sequence set forth in SEQ ID No: 3, the polypeptide (D1) ranging from A2 to N103 thereof, and the polypeptide (D4) ranging from D253 to N362 thereof are arranged in this order from the N-terminal side, and D2, D1 and D4 may be linked to one another by a polypeptide consisting of one to several amino acids. Furthermore, a polypeptide consisting of one to several amino acids may be added to the C-terminal side of D4 or the N-terminal side of D2.

When any one or both of domains constituting a domain-modified body are deleted, it is described such as, for example, D1-D2-D3 (del C 25), D1-D2 (del N 93), D1-D2 (del N 93) (del C 5), etc. D1-D2-D3 (del C 25) indicates a domain-modified body in which the D1, D2 and D3 domains are linked to one another in this order from the N-terminal side, but 25 amino acids are deleted from the C-terminal side of D1-D2-D3. In addition, D1-D2 (del N 93) indicates a domain-modified body in which the D1 and D2 domains are linked to each other in this order from the N-terminal side, but 93 amino acids are deleted from the N-terminal side of D1-D2. Furthermore, D1-D2 (del N 93) (del C 5) indicates a domain-modified body in which the D1 and D2 domains are linked to each other in this order from the N-terminal side, but 93 amino acids are deleted from the N-terminal side of D1-D2, and 5 amino acids are deleted from the C-terminal side. It is to be noted that the designation of the above-described domain-modified bodies refers to either the domain-modified body itself or the amino acid sequence of the domain-modified body.

In the present description, the term "amino acid" is used in the broadest sense, and includes not only natural amino acids, but also their derivatives and artificial amino acids. Examples of the amino acids described in the present description may include, for example, natural amino acids, as well as unnatural amino acids. In addition, examples of the "amino acid" may also include amino acids whose main chain structure differs from that of natural amino acids, amino acids whose side chain structure differs from that of natural amino acids, amino acids having extra methylene in the side chain thereof, and amino acids in which the carboxylic acid functional group in the side chain thereof is substituted with a sulfonic acid group.

The protein set according to the present embodiment includes a set of an RpBphP1 mutant and a wild-type RpPpsR2, a set of a wild-type RpBphP1 and an RpPpsR2 mutant, and a set of an RpBphP1 mutant and an RpPpsR2 mutant.

The RpBphP1 mutant according to the present embodiment includes mutants consisting of amino acid sequences having one or multiple point mutations, in which the following amino acids are substituted with other amino acids, in the amino acid sequence as set forth in SEQ ID No: 1 or SEQ ID No: 2:
E327, E332, E337, D339, E365, Q367, E429, R490, D498, Q502, F503, R504, R507, R512, E513, Q514, E516, F518, Q521, S525, D607, E613, R636, R638, F639, D654, H661, E678, Y681, E693, R696, and H712.

More specifically, the RpBphP1 mutant according to the present embodiment includes mutants consisting of amino acid sequences having any one or multiple point mutations as shown below, in the amino acid sequence as set forth in SEQ ID No: 1 or SEQ ID No: 2:
Q514A, D607E, R638A, H661A, R696A,
E327A, E332A, E337A, D339A, E365A, Q367A, E429A, R490A, D498A, Q502A, Q502G, Q502V, Q502L, Q502I, Q502S, Q502T, Q502E, Q502K, Q502R, Q502H, Q502N, Q502F, Q502Y, Q502W, Q502C, Q502M, Q502P, F503A, R504A, R507A, E513G, E513D, E513P, E516A, F518A, S525A, D607A, D607G, D607V, D607L, D607I, D607S, D607T, D607K, D607R, D607H, D607N, D607Q, D607F, D607W, D607C, D607M, D607P, E613A, R636A, F639A, D654A, E678A, Y681A, E693A, H712D, H712E, H712P,
R512A, E513V, E513A, E513L, E513T, E513M, and Q521A.

The above-described point mutations are classified into the following 3 groups:
(1) mutations, in which the binding of an RpBphP1 mutant having at least one of the present mutations and a wild-type RpPpsR2 is weak under the dark, and is almost equivalent under near-infrared light irradiation, compared to the binding of a wild-type RpBphP1 and a wild-type RpPpsR2:
   Q514A, D607E, R638A, H661A, and R696A;
(2) mutations, in which the binding of an RpBphP1 mutant having at least one of the present mutations and a wild-type RpPpsR2 is weak under the dark, and is weak also under near-infrared light irradiation, compared to the binding of a wild-type RpBphP1 and a wild-type RpPpsR2:
   E327A, E332A, E337A, D339A, E365A, Q367A, E429A, R490A, D498A, Q502A, Q502G, Q502V, Q502L, Q502I, Q502S, Q502T, Q502E, Q502K, Q502R, Q502H, Q502N, Q502F, Q502Y, Q502W, Q502C, Q502M, Q502P, F503A, R504A, R507A, E513G, E513D, E513P, E516A, F518A, S525A, D607A, D607G, D607V, D607L, D607I, D607S, D607T, D607K, D607R, D607H, D607N, D607Q, D607F, D607W, D607C, D607M, D607P, E613A, R636A, F639A, D654A, E678A, Y681A, E693A, H712D, H712E, and H712P; and
(3) mutations, in which the binding of an RpBphP1 mutant having at least one of the present mutations and a wild-type RpPpsR2 is strong under near-infrared light irradiation, and is strong also under the dark, compared to the binding of a wild-type RpBphP1 and a wild-type RpPpsR2:
   R512A, E513V, E513A, E513L, E513T, E513M, and Q521A.

The RpPpsR2 mutant according to the present embodiment includes mutants consisting of amino acid sequences having one or multiple point mutations, in which the following amino acids are substituted with other amino acids, in the amino acid sequence as set forth in SEQ ID No: 3:
K102, N103, Q105, V107, T108, L110, R113, L114, I115, Q119, M121, R123, D124, Y125, W126, R127, R129, E132, T133, R134, R136, L137, V138, F139, and V146.

More specifically, the RpPpsR2 mutant according to the present embodiment includes mutants consisting of amino acid sequences having any one or multiple point mutations as shown below, in the amino acid sequence as set forth in SEQ ID No: 3:
Q105T, T133A, L137A,
N103A, Q105A, Q105C, Q105H, V107A, T108A, L110A, R113A, L114A, I115A, Q119A, M121A, M121E, R123A, D124A, Y125A, Y125E, W126A, R127A, R129A, E132A, R134A, R136A,
Q105V, Q105S,
K102A, Q105P, Q105R, L137E, V138A, V138E, F139E, and V146A.

The above-described point mutations are classified into the following 4 groups:
(1) mutations, in which the binding of an RpPpsR2 mutant having at least one of the present mutations and a wild-type RpBphP1 is weak under the dark, and is almost equivalent under near-infrared light irradiation, compared to the binding of a wild-type RpBphP1 and a wild-type RpPpsR2:
   Q105T, T133A, and L137A;
(2) mutations, in which the binding of an RpPpsR2 mutant having at least one of the present mutations and a wild-type RpBphP1 is weak under the dark, and is weak also under near-infrared light irradiation, compared to the binding of a wild-type RpBphP1 and a wild-type RpPpsR2:
   N103A, Q105A, Q105C, Q105H, V107A, T108A, L110A, R113A, L114A, I115A, Q119A, M121A, M121E, R123A, D124A, Y125A, Y125E, W126A, R127A, R129A, E132A, R134A, and R136A;
(3) mutations, in which the binding of an RpPpsR2 mutant having at least one of the present mutations and a wild-type RpBphP1 is strong under near-infrared light irradiation, and is almost equivalent under the dark, compared to the binding of a wild-type RpBphP1 and a wild-type RpPpsR2:
   Q105V and Q105S; and
(4) mutations, in which the binding of an RpPpsR2 mutant having at least one of the present mutations and a wild-type RpBphP1 is strong under near-infrared light irradiation, and is strong also under the dark, compared to the binding of a wild-type RpBphP1 and a wild-type RpPpsR2:
   K102A, Q105P, Q105R, L137E, V138A, V138E, F139E, and V146A.

Furthermore, the RpPpsR2 mutant according to the present embodiment includes mutants consisting of amino acid sequences, in which the amino acid sequence as set forth in SEQ ID No: 3 is modified to any of the following domain-modified sequences:
D1-D1-D2, D3-D1-D2, D1-D2 (del N 77),
D1-D2, D2-D1, D2-D2, D3-D2, D4-D2, D1-D2-D1, D1-D2-D2, D1-D2-D4, D2-D1-D2, D2-D3-D4, D4-D1-D2, D2-D3-D4-D1, D2-D3-D4-D2, D2-D3-D4-D3, D2-D3-D4-D5, D1-D2 (del N 88), D1-D2 (del N 89), D1-D2 (del N 90), D1-D2 (del N 91), D1-D2 (del N 92), D1-D2 (del N 93), D1-D2 (del N 94), D1-D2 (del N 95), D1-D2 (del N 96), D1-D2 (del N 97), D1-D2 (del C 5), D1-D2 (del C 15), D1-D2 (del N 93) (del C 5), D1-D2 (del N 93) (del C 6), D1-D2 (del N 93) (del C 11), D1-D2 (del N 93) (del C 12), D1-D2-D3 (del C 25), D1-D2-D3 (del C 41), D1-D2-D3 (del C 49), D1-D2-D3 (del C 70), D1-D2-D3 (del C 87), D1-D2-D3 (del C 96), D1-D2-D3 (del C 104),
D1-D2 (del N 64),
D2-D3, D1-D2-D3, D2-D2-D3, D2-D3-D1, D2-D3-D3, D3-D2-D3, D4-D2-D3, D1-D1-D2-D3, D1-D2-D3-D1, D1-D2-D3-D2, D1-D2-D3-D3, D1-D2-D3-D4, D2-D1-D2-D3, D2-D2-D3-D4, D2-D3-D4-D4, D3-D1-D2-D3, D3-D2-D3-D4, D1-D2 (del N 15), D1-D2 (del N 33), D1-D2 (del N 47), and D1-D2-D3 (del C 9).

The above-described domain-modified bodies are classified into the following 4 groups:
(1) mutations, in which the binding of a domain-modified body of the RpPpsR2 and a wild-type RpBphP1 is weak under the dark, and is almost equivalent under near-infrared light irradiation, compared to the binding of a wild-type RpBphP1 and a wild-type RpPpsR2:
   D1-D1-D2, D3-D1-D2, and D1-D2 (del N 77);
(2) mutations, in which the binding of a domain-modified body of the RpPpsR2 and a wild-type RpBphP1 is weak under the dark, and is weak also under near-infrared light irradiation, compared to the binding of a wild-type RpBphP1 and a wild-type RpPpsR2:
   D1-D2, D2-D1, D2-D2, D3-D2, D4-D2, D1-D2-D1, D1-D2-D2, D1-D2-D4, D2-D1-D2, D2-D3-D4, D4-D1-D2, D2-D3-D4-D1, D2-D3-D4-D2, D2-D3-D4-D3, D2-D3-D4-D5, D1-D2 (del N 88), D1-D2 (del N 89), D1-D2 (del N 90), D1-D2 (del N 91), D1-D2 (del N 92), D1-D2 (del N 93), D1-D2 (del N 94), D1-D2 (del N 95), D1-D2 (del N 96), D1-D2 (del N 97), D1-D2 (del C 5), D1-D2 (del C 15), D1-D2 (del N 93) (del C 5), D1-D2 (del N 93) (del C 6), D1-D2 (del N 93) (del C 11), D1-D2 (del N 93) (del C 12), D1-D2-D3 (del C 25), D1-D2-D3 (del C 41), D1-D2-D3 (del C 49), D1-D2-D3 (del C 70), D1-D2-D3 (del C 87), D1-D2-D3 (del C 96), and D1-D2-D3 (del C 104);
(3) a mutation, in which the binding of a domain-modified body of the RpPpsR2 and a wild-type RpBphP1 is strong under near-infrared light irradiation, and is almost equivalent under the dark, compared to the binding of a wild-type RpBphP1 and a wild-type RpPpsR2:
   D1-D2 (del N 64); and
(4) mutations, in which the binding of a domain-modified body of the RpPpsR2 and a wild-type RpBphP1 is strong under near-infrared light irradiation, and is strong also under the dark, compared to the binding of a wild-type RpBphP1 and a wild-type RpPpsR2:
   D2-D3, D1-D2-D3, D2-D2-D3, D2-D3-D1, D2-D3-D3, D3-D2-D3, D4-D2-D3, D1-D1-D2-D3, D1-D2-D3-D1, D1-D2-D3-D2, D1-D2-D3-D3, D1-D2-D3-D4, D2-D1-D2-D3, D2-D2-D3-D4, D2-D3-D4-D4, D3-D1-D2-D3, D3-D2-D3-D4, D1-D2 (del N 15), D1-D2 (del N 33), D1-D2 (del N 47), and D1-D2-D3 (del C 9).

Furthermore, the RpPpsR2 mutant according to the present embodiment also includes mutants having both the above-described point mutation and a mutation caused by domain modification.

That is to say, the RpPpsR2 mutant according to the present embodiment includes mutants consisting of amino acid sequences, in which the amino acid sequence as set forth in SEQ ID No: 3 is modified to any of the following domain modified bodies:
D1-D1-D2, D3-D1-D2, D1-D2 (del N 77),
D1-D2, D2-D1, D2-D2, D3-D2, D4-D2, D1-D2-D1, D1-D2-D2, D1-D2-D4, D2-D1-D2, D2-D3-D4, D4-D1-D2, D2-D3-D4-D1, D2-D3-D4-D2, D2-D3-D4-D3, D2-D3-D4-D5, D1-D2 (del N 88), D1-D2 (del N 89), D1-D2 (del N 90), D1-D2 (del N 91), D1-D2 (del N 92), D1-D2 (del N 93), D1-D2 (del N 94), D1-D2 (del N 95), D1-D2 (del N 96), D1-D2 (del N 97), D1-D2 (del C 5), D1-D2 (del C 15), D1-D2 (del N 93) (del C 5), D1-D2 (del N 93) (del C 6), D1-D2 (del N 93) (del C 11), D1-D2 (del N 93) (del C 12), D1-D2-D3 (del C 25), D1-D2-D3 (del C 41), D1-D2-D3 (del C 49), D1-D2-D3 (del C 70), D1-D2-D3 (del C 87), D1-D2-D3 (del C 96), D1-D2-D3 (del C 104),
D1-D2 (del N 64),
D2-D3, D1-D2-D3, D2-D2-D3, D2-D3-D1, D2-D3-D3, D3-D2-D3, D4-D2-D3, D1-D1-D2-D3, D1-D2-D3-D1, D1-D2-D3-D2, D1-D2-D3-D3, D1-D2-D3-D4, D2-D1-D2-D3, D2-D2-D3-D4, D2-D3-D4-D4, D3-D1-D2-D3, D3-D2-D3-D4, D1-D2 (del N 15), D1-D2 (del N 33), D1-D2 (del N 47), and D1-D2-D3 (del C 9), wherein
the mutants have, in the amino acid sequences thereof, point mutations corresponding to any one or multiple of the following a point mutation(s) in the amino acid sequence as set forth in SEQ ID No: 3:
   Q105T, T133A, L137A,
   N103A, Q105A, Q105C, Q105H, V107A, T108A, L110A, R113A, L114A, I115A, Q119A, M121A, M121E, R123A, D124A, Y125A, Y125E, W126A, R127A, R129A, E132A, R134A, R136A,
   Q105V, Q105S,
   K102A, Q105P, Q105R, L137E, V138A, V138E, F139E, and V146A.

More specifically, the RpPpsR2 mutant having both a domain modification and a point mutation will be described, taking a mutant having both a D3-D2 modification mutation and an L137E point mutation as an example. A D3-D2 modified body also having an L137E point mutation means a D3-D2 modified body, in which the 137th lysine in the amino acid sequence as set forth in SEQ ID No: 3 is substituted with glutamic acid, and this D3-D2 modified body is referred to as "D3-D2 (L137E)" in the present description.

An example of the above-described RpPpsR2 mutant having both a domain modification and a point mutation may be the above-described D3-D2 (L137E), and this is a mutant, in which the binding of the D3-D2 (L137E) and a wild-type RpBphP1 is weak under the dark, and is weak also under near-infrared light irradiation, compared to the binding of a wild-type RpBphP1 and a wild-type RpPpsR2.

The protein set according to the present embodiment includes a protein set of a wild-type RpPpsR2 and an RpBphP1 mutant, wherein
the RpBphP1 mutant is a mutant consisting of an amino acid sequence having one or multiple point mutations selected from the group of point mutations consisting of:
Q514A, D607E, R638A, H661A, R696A
E327A, E332A, E337A, D339A, E365A, Q367A, E429A, R490A, D498A, Q502A, Q502G, Q502V, Q502L, Q502I, Q502S, Q502T, Q502E, Q502K, Q502R, Q502H, Q502N, Q502F, Q502Y, Q502W, Q502C, Q502M, Q502P, F503A, R504A, R507A, E513G, E513D, E513P, E516A, F518A, S525A, D607A, D607G, D607V, D607L, D607I, D607S, D607T, D607K, D607R, D607H, D607N, D607Q, D607F, D607W, D607C, D607M, D607P, E613A, R636A, F639A, D654A, E678A, Y681A, E693A, H712D, H712E, H712P,
R512A, E513V, E513A, E513L, E513T, E513M, and Q521A, in the amino acid sequence as set forth in SEQ ID No: 1 or SEQ ID No: 2.

The protein set according to the present embodiment includes a protein set of a wild-type RpBphP1 and an RpPpsR2 mutant, wherein
the RpPpsR2 mutant is a mutant consisting of an amino acid sequence having one or multiple point mutations selected from the group of point mutations consisting of:
Q105T, T133A, L137A,
N103A, Q105A, Q105C, Q105H, V107A, T108A, L110A, R113A, L114A, I115A, Q119A, M121A, M121E, R123A, D124A, Y125A, Y125E, W126A, R127A, R129A, E132A, R134A, R136A,
Q105V, Q105S,
K102A, Q105P, Q105R, L137E, V138A, V138E, F139E, and V146A, in the amino acid sequence as set forth in SEQ ID No: 3.

The protein set according to the present embodiment includes a protein set of a wild-type RpBphP1 and an RpPpsR2 mutant, wherein
the RpPpsR2 mutant is a mutant consisting of an amino acid sequence, in which the amino acid sequence as set forth in SEQ ID No: 3 is modified to any of:
D1-D1-D2, D3-D1-D2, D1-D2 (del N 77),
D1-D2, D2-D1, D2-D2, D3-D2, D3-D2 (L137E), D4-D2, D1-D2-D1, D1-D2-D2, D1-D2-D4, D2-D1-D2, D2-D3-D4, D4-D1-D2, D2-D3-D4-D1, D2-D3-D4-D2, D2-D3-D4-D3, D2-D3-D4-D5, D1-D2 (del N 88), D1-D2 (del N 89), D1-D2 (del N 90), D1-D2 (del N 91), D1-D2 (del N 92), D1-D2 (del N 93), D1-D2 (del N 94), D1-D2 (del N 95), D1-D2 (del N 96), D1-D2 (del N 97), D1-D2 (del C 5), D1-D2 (del C 15), D1-D2 (del N 93) (del C 5), D1-D2 (del N 93) (del C 6), D1-D2 (del N 93) (del C 11), D1-D2 (del N 93) (del C 12), D1-D2-D3 (del C 25), D1-D2-D3 (del C 41), D1-D2-D3 (del C 49), D1-D2-D3 (del C 70), D1-D2-D3 (del C 87), D1-D2-D3 (del C 96), D1-D2-D3 (del C 104),
D1-D2 (del N 64),
D2-D3, D1-D2-D3, D2-D2-D3, D2-D3-D1, D2-D3-D3, D3-D2-D3, D4-D2-D3, D1-D1-D2-D3, D1-D2-D3-D1, D1-D2-D3-D2, D1-D2-D3-D3, D1-D2-D3-D4, D2-D1-D2-D3, D2-D2-D3-D4, D2-D3-D4-D4, D3-D1-D2-D3, D3-D2-D3-D4, D1-D2 (del N 15), D1-D2 (del N 33), D1-D2 (del N 47), and D1-D2-D3 (del C 9).

The protein set according to the present embodiment includes a protein set of an RpBphP1 mutant and an RpPpsR2 mutant, wherein
the RpBphP1 mutant is a mutant consisting of an amino acid sequence having one or multiple point mutations selected from the group of point mutations consisting of:
Q514A, D607E, R638A, H661A, R696A,
E327A, E332A, E337A, D339A, E365A, Q367A, E429A, R490A, D498A, Q502A, Q502G, Q502V, Q502L, Q502I, Q502S, Q502T, Q502E, Q502K, Q502R, Q502H, Q502N, Q502F, Q502Y, Q502W, Q502C, Q502M, Q502P, F503A, R504A, R507A, E513G, E513D, E513P, E516A, F518A, S525A, D607A, D607G, D607V, D607L, D607I, D607S, D607T, D607K, D607R, D607H, D607N, D607Q, D607F, D607W, D607C, D607M, D607P, E613A, R636A, F639A, D654A, E678A, Y681A, E693A, H712D, H712E, H712P,
R512A, E513V, E513A, E513L, E513T, E513M, and Q521A, in the amino acid sequence as set forth in SEQ ID No: 1 or SEQ ID No: 2, and
the RpPpsR2 mutant is any mutant consisting of an amino acid sequence having one or multiple point mutations selected from the group of point mutations consisting of:
   Q105T, T133A, L137A,
   N103A, Q105A, Q105C, Q105H, V107A, T108A, L110A, R113A, L114A, I115A, Q119A, M121A, M121E, R123A, D124A, Y125A, Y125E, W126A, R127A, R129A, E132A, R134A, R136A,
   Q105V, Q105S,
   K102A, Q105P, Q105R, L137E, V138A, V138E, F139E, and V146A, in the amino acid sequence as set forth in SEQ ID No: 3.

The protein set according to the present embodiment includes a protein set of an RpBphP1 mutant and an RpPpsR2 mutant, wherein
the RpBphP1 mutant is any mutant consisting of an amino acid sequence having one or multiple point mutations selected from the group of point mutations consisting of:
Q514A, D607E, R638A, H661A, R696A,
E327A, E332A, E337A, D339A, E365A, Q367A, E429A, R490A, D498A, Q502A, Q502G, Q502V, Q502L, Q502I, Q502S, Q502T, Q502E, Q502K, Q502R, Q502H, Q502N, Q502F, Q502Y, Q502W, Q502C, Q502M, Q502P, F503A, R504A, R507A, E513G, E513D, E513P, E516A, F518A, S525A, D607A, D607G, D607V, D607L, D607I, D607S, D607T, D607K, D607R, D607H, D607N, D607Q, D607F, D607W, D607C, D607M, D607P, E613A, R636A, F639A, D654A, E678A, Y681A, E693A, H712D, H712E, H712P,
R512A, E513V, E513A, E513L, E513T, E513M, and Q521A, in the amino acid sequence as set forth in SEQ ID No: 1 or SEQ ID No: 2, and
the RpPpsR2 mutant is a mutant consisting of an amino acid sequence, in which the amino acid sequence as set forth in SEQ ID No: 3 is modified to any of:
   D1-D1-D2, D3-D1-D2, D1-D2 (del N 77),
   D1-D2, D2-D1, D2-D2, D3-D2, D3-D2 (L137E), D4-D2, D1-D2-D1, D1-D2-D2, D1-D2-D4, D2-D1-D2, D2-D3-D4, D4-D1-D2, D2-D3-D4-D1, D2-D3-D4-D2, D2-D3-D4-D3, D2-D3-D4-D5, D1-D2 (del N 88), D1-D2 (del N 89), D1-D2 (del N 90), D1-D2 (del N 91), D1-D2 (del N 92), D1-D2 (del N 93), D1-D2 (del N 94), D1-D2 (del N 95), D1-D2 (del N 96), D1-D2 (del N 97), D1-D2 (del C 5), D1-D2 (del C 15), D1-D2 (del N 93) (del C 5), D1-D2 (del N 93) (del C 6), D1-D2 (del N 93) (del C 11), D1-D2 (del N 93) (del C 12), D1-D2-D3 (del C 25), D1-D2-D3 (del C 41), D1-D2-D3 (del C 49), D1-D2-D3 (del C 70), D1-D2-D3 (del C 87), D1-D2-D3 (del C 96), D1-D2-D3 (del C 104),
   D1-D2 (del N 64),
   D2-D3, D1-D2-D3, D2-D2-D3, D2-D3-D1, D2-D3-D3, D3-D2-D3, D4-D2-D3, D1-D1-D2-D3, D1-D2-D3-D1, D1-D2-D3-D2, D1-D2-D3-D3, D1-D2-D3-D4, D2-D1-D2-D3, D2-D2-D3-D4, D2-D3-D4-D4, D3-D1-D2-D3, D3-D2-D3-D4, D1-D2 (del N 15), D1-D2 (del N 33), D1-D2 (del N 47), and D1-D2-D3 (del C 9).

The binding powder between proteins constituting the photoswitching proteins according to the present embodiment (i.e., an RpBphP1 mutant and a wild-type RpPpsR2, a wild-type RpBphP1 and an RpPpsR2 mutant, and an RpBphP1 mutant and an RpPpsR2 mutant) can be easily measured by those skilled in the art by using a method well known in the present technical field, or a modified method of the well-known method. Such a method may be, for example, a method utilizing CPTS disclosed in the Examples.

Hereafter, the operating principle of CPTS (CRISPR-Cas9-based photoactivatable transcription system) will be described. CPTS is a light manipulation tool that utilizes Cas9 lacking DNA cleavage activity. CPTS can be used for activation of a genome gene or as a reporter assay system. Cas9 mutants that lack DNA cleavage activity (e.g., Cas9D10A/H840A; dCas9) can form a complex with guide RNA and can bind to the DNA nucleotide sequence that is targeted by the guide RNA. Among several loop structures of guide RNA, when an aptamer sequence that binds to a specific protein (e.g., "Protein 1") is inserted into a loop exposed from dCas9, Protein 1 can bind to dCas9 via the aptamer sequence.

One protein (for example, a "wild-type RpBphP1") of the photoswitching proteins according to the present embodiment is linked to Protein 1, and a transcriptional activator (for example, p65, HSF1, etc.) is linked to the other protein (for example, an "RpPpsR2 mutant") of the photoswitching proteins according to the present embodiment. That is to say, a linked body of Protein 1-wild-type RpBphP1 and a linked body of RpPpsR2 mutant-transcriptional activator are prepared. In a state in which a reporter gene that has the nucleotide sequence of DNA targeted by guide RNA near the transcription start site, a complex of the linked body of Protein 1-wild-type RpBphP1 and dCas9, and the linked body of RpPpsR2 mutant-transcriptional activator coexist, dCas9 binds near the nucleotide sequence of the DNA targeted by the guide RNA.

Here, when a light (in the present embodiment, a near-infrared light) is irradiated, the wild-type RpBphP1 and the RpPpsR2 mutant bind to each other, and the transcriptional activator linked to the RpPpsR2 mutant is anchored near the transcription start point. As a result, transcription of the reporter gene is activated, mRNA transcription occurs, and translation into a protein occurs. On the other hand, when the light irradiation is terminated, since the wild-type RpBphP1 and the RpPpsR2 mutant dissociate from each other, the transcriptional activator is largely separated from the transcription start point, and the expression of the gene is terminated.

Using the above-mentioned CPTS, the binding power of the RpBphP1 mutant and the wild-type RpPpsR2, the wild-type RpBphP1 and the RpPpsR2 mutant, or the RpBphP1 mutant and the RpPpsR2 mutant can be quantitatively evaluated using the expression level of the reporter gene as an indicator.

In addition to the method utilizing CPTS, the binding power of proteins that constitute the photoswitching proteins according to the present embodiment can also be measured by a method utilizing a reporter gene assay using TetO-tetR, a fluorescent microscope assay using translocation to the cell membrane as an indicator, and the like.

Individual proteins that constitute the photoswitching proteins according to the present embodiment (e.g., a wild-type RpBphP1 and an RpPpsR2 mutant) may be bound to or fused with "other substances (i.e., substances different from individual proteins that constitute the photoswitching proteins)." Such "other substances" are not particularly limited, and may be any substances. Examples of such other substances may include various biological materials such as proteins, lipids, sugars, and nucleic acids. The photoswitching proteins according to the present embodiment and "other substances" may be bound to one another via linkers or the like, and when the "other substances" are proteins, they may be fused with one another.

In the above-mentioned explanation of CPTS, "Protein 1" and "transcriptional activator" correspond to "other substances."

Alternatively, as such "other substances," interacting Protein A and Protein B may be bound to or fused with individual proteins of the proteins of the photoswitching proteins according to the present embodiment. In this case, when the photoswitching proteins according to the present embodiment are irradiated with a near-infrared light, the interaction between Protein A and Protein B can be induced, and when the near-infrared light irradiation is terminated, Protein A and Protein B can be dissociated from each other. Therefore, by using the photoswitching proteins according to the present embodiment, it is possible to freely control the interaction between proteins by turning light irradiation ON/OFF. In addition to the examples shown herein, there are other useful intended uses of the photoswitching proteins according to the present embodiment (see Non Patent Literature 1 and Non Patent Literature 2, etc.).

A second embodiment relates to a nucleic acid (DNA or RNA) that encodes individual proteins constituting the photoswitching proteins according to the present embodiment, namely, the RpBphP1 mutant and the RpPpsR2 mutant.

The nucleic acid according to the present embodiment can be prepared by a method known in the present technical field, or a method obtained by appropriately modifying the method. For example, a desired nucleic acid may be obtained by introducing a desired mutation into DNA that encodes the wild type. Alternatively, the present nucleic acid can also be synthesized by an automatic synthesizer. When individual proteins that constitutes the photoswitching proteins according to the present embodiment are fused with other proteins, the nucleic acids that encode such fusion proteins are also included in the nucleic acid according to the present embodiment.

The wild-type RpBphP1, wild-type RpPpsR2, RpBphP1 mutant and RpPpsR2 mutant according to the present embodiment can be prepared by incorporating nucleic acids encoding them into suitable expression vectors, respectively, then transforming or transfecting suitable host cells with the expression vectors, then culturing the host cells in an appropriate medium, and then purifying each of the expressed proteins.

Examples of host cells for protein expression that may be used herein may include bacterial cells (e.g., Escherichia coli B strain, E. coli Kl2 strain, Corynebacterium ammoniagenes, C. glutamicum, Serratia liquefaciens, Streptomyces lividans, Pseudomonas putida, etc.), molds (e.g., Penicillium camembertii, Acremonium chrysogenum, etc.), animal cells, plant cells, baculovirus/insect cells, and yeast cells (e.g., Saccharomyces cerevisiae and Pichia pastoris, etc.).

As an expression vector for protein expression, a vector suitable for various types of host cells can be used. Examples of the expression vector that can be used herein may include: pBR322, pBR325, pUC118, pET, etc. (for E. coli hosts); pEGFP-C, pEGFP-N, etc. (for animal cell hosts); pVL1392, pVL1393, etc. (for insect cell hosts; baculovirus vectors); and pG-1, Yep13, pPICZ, etc. (for yeast cell hosts). These expression vectors may comprise a replication origin, a selection marker, and a promoter, which are suitable for each of these vectors, and the vectors may also comprise, if necessary, an enhancer, a transcription termination sequence (terminator), a ribosome-binding site, a polyadenylation signal, and the like. Furthermore, in order to facilitate purification of the expressed polypeptide, a nucleotide sequence for fusing and expressing a FLAG tag, a His tag, an HA tag, a GST tag, etc. may be inserted into the expression vector.

The expression vector can be prepared by a method known to those skilled in the art, and can also be prepared using a commercially available kit, etc., as appropriate.

When the expressed protein is extracted from cultured cell masses or cultured cells, the cell masses or the cultured cells are collected by a known method after completion of the culture, and the collected cell masses or cells are then suspended in a suitable buffer. Thereafter, the suspension is subjected to ultrasonic wave, lysozyme and/or freezing-thawing, etc., so that the cell masses or cells are disintegrated. Thereafter, the resultant is subjected to centrifugation or filtration to obtain a soluble extract. In particular, when cultured cells are used as hosts, it is desirable to obtain the protein expressed in a culture supernatant by recovering the supernatant. An appropriate combination of known separation and/or purification methods is applied to the obtained extract or culture supernatant, so that a protein of interest can be obtained. Examples of the known separation and/or purification methods that is used herein may include: methods of utilizing solubility, such as salting-out or a solvent precipitation method; methods of mainly utilizing a difference in molecular weights, such as a dialysis method, an ultrafiltration method, a gel filtration method, or SDS-PAGE; methods of utilizing a difference in electric charges, such as ion exchange chromatography; methods of utilizing specific affinity, such as affinity chromatography (for example, methods, in which when a polypeptide is expressed together with a GST tag, a glutathione-bound carrier resin is used, when a polypeptide is expressed together with a His tag, a Ni-NTA resin or a Co-based resin is used, when a polypeptide is expressed together with a HA tag, an anti-HA antibody resin is used, and when a polypeptide is expressed together with a FLAG tag, an anti-FLAG antibody-bound resin or the like is used); methods of utilizing a difference in hydrophobicity, such as reverse phase high performance liquid chromatography; and methods of utilizing a difference in isoelectric points, such as an isoelectric focusing method.

When the present description is translated into English and the English description includes singular terms with the articles "a," "an," and "the," these terms include not only single items but also multiple items, unless otherwise clearly specified from the context that it is not the case.

Hereinafter, the present invention will be further described in the following examples. However, these examples are only illustrative examples of the embodiments of the present invention, and thus, are not intended to limit the scope of the present invention.

### Examples

### 1. Experimental methods

### 1-1. Wild-type RpBphP1 gene and wild-type RpPpsR2 gene

The wild-type RpBphP1 gene (SEQ ID No: 4) and the wild-type RpPpsR2 gene (SEQ ID No: 5) were purchased from the Artificial Gene Synthesis Services of GenScript. At that time, the genes were optimized for human codons, using the codon optimization algorithm of GenScript.

### 1-2. Introduction of point mutations

Point mutations were introduced into RpBphP1 and RpPpsR2, using an overlap extension PCR method (Ho, S. N et al., Gene 77: 51-59, 1989) or a transfer PCR method (Erijman et al., J. Struct. Biol. 175:171-177, 2011). For both of the PCR methods, oligo DNAs used as primers were purchased from Eurofins Genomics. As DNA polymerase, PrimeSTAR HS DNA polymerase from Takara Bio, Inc. was used, and the PCR reaction conditions were as described in the product's instruction manual. In the case of point mutation introduction by the overlap extension PCR method, the obtained PCR product fragment was treated with restriction enzymes and was then ligated to a vector that had been similarly treated with restriction enzymes. Restriction enzymes from Takara Bio, Inc. or from New England Bio were used, and Ligation high Ver. 2 manufactured by TOYOBO Co., Ltd. was used for the ligation reaction. The reaction conditions for each reaction were as described in the product's instruction manual. As a vector, pcDNA3.1-V5HisA, a mammalian cell expression vector manufactured by Invitrogen, was used. The ligation product was mixed with Mach1 chemically competent cells of Invitrogen, and was transformed by heat shock at 42°C. Plasmids were cloned from the transformed E. coli according to standard methods for genetic engineering experiments. The sequences of the obtained plasmids were confirmed by the Sequencing Service of Eurofins Genomics. In the case of point mutation introduction by the transfer PCR method, a plasmid was first prepared by cloning a wild-type sequence into pcDNA3.1-V5HisA, and a transfer PCR reaction was then carried out using this plasmid as a template. After completion of the PCR reaction, the wild-type template plasmid was digested with Dpn I of Takara Bio, Inc. The product obtained after the digestion reaction was transformed into Mach1 chemically competent cells, and was cloned according to standard methods for genetic engineering experiments. The sequence of the obtained plasmid was confirmed by the Sequencing Service of Eurofins Genomics.

### 1-3. Domain shuffling

Domain shuffling of RpPpsR2 was carried out as follows. First, a restriction enzyme site was added to a domain to be shuffled. For this, using primers that had been designed to include restriction enzyme sites, PCR amplification was carried out according to a standard PCR method. The obtained domain fragments were cloned into pcDNA3.1-V5HisA, and their sequences were then confirmed by the Sequencing Service of Eurofins Genomics. The sequence-confirmed domain fragments were cleaved by a restriction enzyme treatment, were then ligated in a desired combination, and were then cloned into pcDNA3.1-V5HisA.

### 1-4. Preparation of domain-deleted body

A domain-deleted body of RpPpsR2 was prepared as follows according to a standard PCR method. In order to delete the N-terminal side or C-terminal side of a desired domain, primers were designed to cause a deletion at the 5'- or 3'-terminal side of the domain DNA. The deleted domain was amplified by a standard PCR method. The resulting deleted domain fragment was cloned into pcDNA3.1-V5HisA, and the sequence was then confirmed by the Sequencing Service of Eurofins Genomics.

### 1-5. Introduction of mutants into CPTS system

The functions of mutants were evaluated using a light-dependent gene activation tool based on the CRISPR-Cas9 system (CRISPR-Cas9-based photoactivatable transcription system; CPTS). CPTS is a tool developed by the present inventors (Nihongaki et al., Nature Methods 14: 963-966, 2017). The CPTS system consists of the following four components: (1) a mutant in which mutations are introduced into the 10th and 840th amino acids of Cas9 to delete DNA cleavage activity (Cas9D10A/H840A; dCas9), (2) guide RNA, into which an aptamer sequence that binds to an MS2 coat protein has been inserted, (3) one of a photoswitching protein pair linked to the MS2 coat protein, and (4) the other of the photoswitching protein pair linked to a transcriptional activator (p65-HSF1). Since the photoswitching protein pair used in the present study consisted of an RpBphP1 mutant and an RpPpsR2 mutant, the RpBphP1 mutant was linked to p65-HSF1, and the RpPpsR2 mutant was linked to the MS2 coat protein. The DNA of the p65-HSF1 was amplified by PCR from Addgene plasmid #61423 purchased from Addgene, and was then subcloned, and thereafter, the sequence was confirmed by the Sequencing Service of Eurofins Genomics. The DNA of the MS2 coat protein (MS2 delta FG) was amplified by PCR from Addgene plasmid #27122 purchased from Addgene, and was then subcloned, and thereafter, the sequence was confirmed by the Sequencing Service of Eurofins Genomics. All of these were linked to the photoswitching proteins, using restriction enzyme sites. In addition, to the N-terminus of each construct, a sequence consisting of three consecutive SV40-derived nuclear localization signals (NLS) (NLSx3; ASPKKKRKVEASASPKKKRKVEASASPKKKSKVEAS; SEQ ID No: 4) was linked. The linking of the nuclear localization signals was also carried out using a restriction enzyme site. The resulting NLSx3-RpBphP1 mutant-p65-HSF1 and NLSx3-MS2-RpPpsR2 mutant were cloned into pcDNA3.1-V5HisA.

### 1-6. Preparation of other components of CPTS system

### (1) Preparation of dCas9

As DNA of Streptococcus pyogenes-derived Cas9 (SpCas9) optimized for human codons, hSpCas9 of Addgene plasmid #42230 purchased from Addgene was used. In order to delete the DNA cleavage activity of hSpCas9, point mutations of D10A and H840A were introduced using Quick change Multi Site-Directed Mutagenesis Kit manufactured by Agilent according to the instruction manual thereof. In addition, SV40-derived nuclear localization signal (NLS) was added to each of the N-terminus and C-terminus by PCR. The thus obtained NLS-dSpCas9-NLS was cloned into pcDNA3.1-V5HisA, and the sequence was then confirmed by the Sequencing Service of Eurofins Genomics.

### (2) Preparation of aptamer-added guide RNA

In order to allow mammalian cells to express guide RNA, a pSPgRNA vector (Addgene plasmid #47108) was purchased from Addgene and was then used. An aptamer sequence that binds to an MS2 coat protein was purchased from the Artificial Gene Synthesis Services of Eurofins Genomics. The synthesized aptamer sequence was cloned into the Kpn I/Sac I site of the pSPgRNA vector. A sequence targeting the upstream activation sequence of the GAL4/UAS system was inserted into the Bbs I site of the prepared vector. This target sequence was prepared by annealing oligo DNA purchased from Eurofins Genomics. The sequence of the completed aptamer-added guide RNA was confirmed by the Sequencing Service of Eurofins Genomics.

### 1-7. Preparation of luminescence reporter plasmid

In order to easily evaluate the functions of mutants, an increase or decrease in luminescence signal was used as an indicator. A reporter plasmid, in which a luminescence reporter gene is activated by CPTS in a light irradiation-dependent manner, was used therefor. Using pGL4.31 of Promega, which includes the upstream activation sequence of the GAL4/UAS system, a luminescence reporter gene downstream thereof was used by replacing a short-lived firefly luciferase gene with a normal-lived firefly luciferase gene. The aptamer-added guide RNA as mentioned above was designed to bind to this upstream activation sequence.

### 1-8. Cell culture

The functions of mutants were evaluated using HEK293T cells. HEK293T cells were obtained from the American Type Culture Collection (ATCC). As a medium, there was used Dulbecco's Modified Eagle Medium (DMEM, Sigma Aldrich) supplemented with 10% FBS (Gibco), 100 unit/mL penicillin and 100 µg/mL streptomycin (Gibco), and 0.584 g/L L-glutamine (Gibco). The cells were cultured in the Dulbecco's Modified Eagle Medium at 37°C in a 5% CO₂ incubator.

### 1-9. Irradiation with near-infrared light

An LED light source (760 ± 20 nm) of CCS was used as a light source for a near-infrared light. The irradiation intensity was set to be 10 W/m². In order to prevent a temperature increase in the inside of the incubator and the cell culture plate, a near-infrared light was irradiated in an ON/OFF cycle of 1 min/4 min, using an ON/OFF timer (WT-03N) of CUSTOM. In addition, a light diffusion film (Scotchcal permeability: 30%) of 3M was used for the purpose of irradiating the cell culture plate with the near-infrared light at a uniform intensity.

### 1-10. Luminescence reporter assay of CPTS

### (1) Cell seeding

First, HEK293T cells were seeded on a 96-well black-walled plate (Greiner Bio). 100 µL each of the cell suspension was added to each well, so that the cell concentration became 2.3 x 10⁴ cells/well. When Biliverdin (BV; Frontier Scientific) was added, it was added to a final concentration of 25 µM. Since BV is easily decomposed by a light, the operation was performed in the dark. The plate on which the cells had been seeded was cultured for 24 hours at 37°C in a 5% CO₂ incubator.

### (2) Transfection of plasmids

Transfection of plasmids was carried out by applying either method (A) or (B) below.

### (A) Transfection by lipofection method

Lipofectamine 3000 (Invitrogen) was used as a gene transfection reagent, and the plasmids to be used in the experiment were transfected according to a lipofection method. In accordance with the instruction manual, Lipofectamine was used at 0.2 µL/well, and P3000 was used at 0.2 µL/well. The total amount of DNA transfected was set to be 0.1 µg per well. Plasmids encoding dSpCas9, guide RNA, MS2-RpPpsR2, RpBphP1-p65HSF1, and GAL4UAS-Firefly Luciferase reporter were transfected at a ratio of 1 : 1 : 1 : 1 : 1. In addition, as a negative control, a pcDNA3.1 V5/His-A vector and a plasmid encoding the GAL4UAS-Firefly Luciferase reporter were transfected at a ratio of 4 : 1. After completion of the gene transfection, the plate was immediately wrapped with an aluminum foil for light shading, and was then returned to the incubator.

### (B) Transfection using cationic polymer (PEI MAX)

PEI MAX (Transfection Grade Linear Polyethylenimine Hydrochloride; Polysciences) was used as a gene transfection reagent. PEI MAX was used at 0.6 µL/well, and the plasmids to be used in the experiment were transfected. The total amount of DNAs transfected was set to be 0.1 µg per well. Plasmids each encoding SpCas9, guide RNA, MS2-RpPpsR2, RpBphP1-p65HSF1, and GAL4UAS-Firefly Luciferase reporter were transfected at a ratio of 1 : 1 : 1 : 1 : 1. In addition, as a negative control, a pcDNA3.1 V5/His-A vector and a plasmid encoding the GAL4UAS-Firefly Luciferase reporter were transfected at a ratio of 4 : 1. After completion of the gene transfection, the plate was immediately wrapped with an aluminum foil for light shading, and was then returned to the incubator.

### (3) Near-infrared light irradiation

Twenty-four hours after the gene transfection, 100 µL each of the medium was added to each well to prevent drying due to evaporation of the medium. To BV(+) wells, the medium containing 25 µM BV was added. At this time, operations on both a plate for light irradiation and a plate for dark conditions were performed in the dark. After the replenishing of the medium, the plate for dark conditions was immediately wrapped with an aluminum foil again for light shading, and was left at rest in the incubator. The plate for light irradiation was irradiated with a near-infrared light in the incubator.

### (4) Measurement of luminescence intensity of luminescence reporter gene

Twenty-four hours after initiation of the light irradiation, both plates were recovered. The medium was removed from each well of the recovered plates, and 100 µL each of HBSS (Gibco) containing 200 µM D-Luciferin (FUJIFILM Wako Pure Chemical Corporation) was added to each well. After 30 minutes of incubation at room temperature, bioluminescence was measured using a luminescence plate reader (Centro XS3 LB 960 Plate Reader; Berthold Technologies). The exposure time was set to be 1 second/well.

### 1-11. Confirmation of genome gene activation by the photoswitching proteins of the present invention in cells and in vivo

### 1-11-1. Preparation of aptamer-added guide RNA targeting genome genes

In order to target genome genes, the following sequences were inserted into the Bbs I site of the aptamer-added guide RNA, respectively.
When targeting a human ASCL1 gene: GCAGCCGCTCGCTGCAGCAG (SEQ ID No: 7)
When targeting a human HBG1 gene: GGCTAGGGATGAAGAATAAA (SEQ ID No: 8)
When targeting a human MYOD1 gene: GCCTGGGCTCCGGGGCGTTT (SEQ ID No: 9)
When targeting a human IL1R2 gene: GTCCTTGGCCACTTCCCCATC (SEQ ID No: 10)
When targeting a mouse ASCL1 gene: AGCTGAGGAGGTGGGGGAAG (SEQ ID No: 11)

These target sequences were prepared by annealing oligo DNA purchased from Eurofins Genomics. The sequence of the completed aptamer-added guide RNA was confirmed by the Sequencing Service of Eurofins Genomics.

### 1-11-2. Intracellular genome gene activation by CPTS

### (1) Cell seeding

First, HEK293T cells were seeded on a 96-well plate (Thermo Fisher Scientific). 100 µL each of the cell suspension was added to each well, so that the cell concentration became 2.3 x 10⁴ cells/well. When BV was added, it was added to a final concentration of 25 µM. Since BV is easily decomposed by a light, the operation was performed in the dark. The plate on which the cells had been seeded was cultured for 24 hours at 37°C in a 5% CO₂ incubator.

### (2) Transfection of plasmids

The combination of an RpBphP1 mutant (with Q502A and E513A introduced) and a wild-type RpPpsR2 was used as photoswitching proteins. Lipofectamine 3000 (Invitrogen) was used as a gene transfection reagent, and the plasmids to be used in the experiment were transfected according to a lipofection method. In accordance with the instruction manual, Lipofectamine was used at 0.2 µL/well, and P3000 was used at 0.2 µL/well. The total amount of DNA transfected was set to be 0.1 µg per well. Plasmids encoding dSpCas9, guide RNA, MS2-RpPpsR2, and RpBphP1-p65HSF1 were transfected at a ratio of 1 : 1 : 1 : 1. In addition, as a negative control, a guide RNA plasmid without having a target sequence was used. After completion of the gene transfection, the plate was immediately wrapped with an aluminum foil for light shading, and was then returned to the incubator.

### (3) Near-infrared light irradiation

Twenty-four hours after the gene transfection, 100 µL each of the medium was added to each well to prevent drying due to evaporation of the medium. To BV(+) wells, the medium containing 25 µM BV was added. At this time, operations on both a plate for light irradiation and a plate for dark conditions were performed in the dark. After the replenishing of the medium, the plate for dark conditions was immediately wrapped with an aluminum foil again for light shading, and was left at rest in the incubator. The plate for light irradiation was irradiated with a near-infrared light in the incubator.

### (4) Evaluation of expression level of genome genes by real-time PCR

Twenty-four hours after initiation of the light irradiation, both plates were recovered. From the cells in the recovered plate, mRNA was extracted using CellAmp Direct RNA Prep Kit manufactured by Takara Bio, Inc. The extraction procedures were according to the instruction manual. Subsequently, the extracted mRNA was reverse-transcribed to cDNA, using Superscript IV VILO Master Mix (Thermo Fisher Scientific). The procedures were according to the instruction manual. After completion of the reverse transcription, the amount of cDNA was evaluated by real-time PCR. As real-time PCR reagents, TaqMan Gene Expression Master Mix (Thermo Fisher Scientific) and TaqMan Gene Expression Assay (Thermo Fisher Scientific) were used. The IDs of the TaqMan probes used are as follows:
human ASCL1 gene: Hs04187546_g1,
human HBG1 gene: Hs00361131_g1,
human MYOD1 gene: Hs02330075_g1,
human IL1R2 gene: Hs01030384_m1, and
human GAPDH gene: Hs99999905_m1.

The real-time PCR was performed, using a thermal cycler for real-time PCR and the software StepOnePlus system (Thermo Fisher Scientific). A standard ΔΔCt method was used in the measurement, and the relative expression level of mRNA to the mRNA of untransfected HEK293T cells was measured.

### 1-11-3. Gene activation in living mouse bodies by CPTS

### (1) Preparation of experimental mice

Animal experiments using mice were conducted in accordance with the Animal Experiment Manual and Experimental Guidelines established by the University of Tokyo. Six-week-old female BALB/c mice (Sankyo Labo Service Corporation, Inc.) were used.

### (2) Gene transfection into mouse liver

The combination of an RpBphP1 mutant (with Q502A and E513A introduced) and a wild-type RpPpsR2 was used as photoswitching proteins. Gene transfection into mouse liver cells was performed by a hydrodynamic tail vein injection method. TransIT-EE Hydrodynamic Delivery Solution manufactured by Mirus Bio was used, and procedures for preparation of the solution were in accordance with the instruction manual. The total amount of plasmid DNAs was set to be 150 µg per mouse. With regard to the ratio of the plasmid DNAs for gene transfection, dSpCas9, guide RNA, MS2-RpPpsR2, RpBphP1-p65HSF1, and GAL4UAS-Akaluc reporter were set to be a ratio of 1: 1 : 1 : 1 : 1. Using a 27G injection needle of Terumo, the prepared solution was injected into the tail vein of mice. At this time, attention was paid to the point that the injection should be completed in about 5 to 6 seconds. After the injection, the mice were returned to their cages and were bred for 8 hours.

### (3) Near-infrared light irradiation to mice

Eight hours after the injection, near-infrared light irradiation to the mice was initiated (Figure 22a). A near-infrared light was irradiated at 760±20 nm and 300 W/m² intensity. Twelve hours after the light irradiation, irradiation was temporarily suspended, and the cage was cleaned.

### (4) Bioluminescence imaging of mouse liver cells

Bioluminescence imaging of mouse liver cells was performed 25 hours after the injection. The mice were intraperitoneally injected with 200 µL of 10 mM Akalumine-HCl (FUJIFILM Wako Pure Chemical Corporation). After the injection, the mice were anesthetized with isoflurane (FUJIFILM Wako Pure Chemical Corporation). After the anesthesia, bioluminescence images of the mice were taken using a Lumazone bioluminescence imager (Shoshin Co., Ltd.) equipped with an Evolve 512 EMCCD camera (Photometrics). Data were analyzed using SlideBook 4.2 software (Intelligent Innovations).

### (5) Genome gene activation in mouse liver cells

Gene transfection was performed by a hydrodynamic tail vein injection method, using guide RNA targeting mouse ASCL1 gene, with dSpCas9, guide RNA, MS2-RpPpsR2, and RpBphP1-p65HSF1 at a ratio of 1 : 1 : 1 : 1. The total amount of plasmid DNA was set to be 120 µg per mouse. Light irradiation was performed in the same manner as that in the case of the aforementioned luminescence reporter gene. After the light irradiation, mRNA was extracted from mouse liver. The mouse liver was disrupted using a Precellys Evolution tissue homogenizer (Bertin Instruments). The extracted mRNA was reverse transcribed to cDNA, using Superscript IV VILO Master Mix (Thermo Fisher Scientific). The procedures were as described in the instruction manual. After the reverse transcription, the amount of cDNA was evaluated by real-time PCR. Luna Universal Probe qPCR Master Mix (New England Biolabs) and TaqMan Gene Expression Assay (Thermo Fisher Scientific) were used as reagents for the real-time PCR. The IDs of the used TaqMan probes were as follows:
mouse ASCL1 gene: Mm03058063_m1; and
mouse GAPDH gene: Mm99999915_g1.

A thermal cycler for real-time PCR, and as software, StepOnePlus system (Thermo Fisher Scientific) were used. A standard ΔΔCt method was used to measure the relative mRNA expression level to the mRNA of untransfected mouse liver cells.

### 2. Results

### 2-1. Confirmation of functions of photoswitching proteins consisting of wild-type RpBphP1 and wild-type RpPpsR2

A wild-type RpBphP1 and a wild-type RpPpsR2 were loaded onto a light-dependent gene activation tool (CPTS) based on the CRISPR-Cas9 system. The operating principle of CPTS is explained below (see Figure 1a).

A mutant (Cas9D10A/H840A; dCas9), in which DNA cleavage activity was lost by introducing mutations into the 10th and 840th amino acids of Cas9, can form a complex with guide RNA and can bind to the nucleotide sequence of genomic DNA targeted by the guide RNA. For example, the target sequence is about 100 to 500 bp upstream of the transcription start point of a gene whose expression is to be light-manipulated. This guide RNA has been somewhat designed. That is, among several loop structures of the guide RNA, into a loop exposed from dCas9, another RNA sequences can be inserted. Thus, an aptamer sequence that binds to a specific protein was inserted therein. In the present experiment, an aptamer sequence that binds to the coat protein possessed by bacteriophage MS2 was inserted. Then, one of the photoswitching proteins is linked to the MS2 coat protein. To the other photoswitching protein, transcriptional activators (p65 and HSF1) were linked. By doing so, when a light is irradiated, the photoswitching proteins bind to each other, and as a result, the transcriptional activators are anchored near the transcription start point. Thereby, transcription of the gene is activated, mRNA transcription occurs, and translation into a protein occurs. On the other hand, when light irradiation is stopped, since the photoswitching proteins are dissociated from each other, the transcriptional activators largely move away from the transcription start point, and the expression of the gene is terminated. In this way, genes encoded on the genome or luminescence reporter genes on luminescence reporter plasmids can be switched ON/OFF by a light.

It was found that a wild-type RpBphP1 and a wild-type RpPpsR2 bind to each other even under the dark and activate genes (WT in Figure 1b). Because these "Dark leak signals" (WT "Dark" in Figure 1b) are extremely high, gene activation by light irradiation increases by only 1.8 times. The occurrence of such "Dark leak signals" is a fatal disadvantage as photoswitching proteins, and they cannot be used as are in practical applications.

### 2-2. Functional analysis of photoswitching proteins consisting of RpBphP1 mutant and wild-type RpPpsR2

An RpBphP1 mutant was prepared by introducing point mutations into RpBphP1, and then, how to change the binding state of the RpBphP1 mutant and a wild-type RpPpsR2 under the dark and under near-infrared light irradiation was examined. As a result, mutants that reduce Dark leak signals compared to a wild-type RpBphP1 (WT) were found (Figures 2a, b and c). Mutants that significantly reduced Dark leak signals could significantly increase gene activation by light irradiation. For example, a D607A mutant increased the gene activation by light irradiation by 15.7 times (Figure 2a), a Q502T mutant increased by 12.4 times (Figure 2b), and a D607S mutant increased by 9.7 times (Figure 2c), respectively. However, these mutants tended to decrease the signals under near-infrared light irradiation (NIR signals) accordingly. On the other hand, such amino acid mutants as D607E, which have the effect of reducing Dark leak signals without reducing NIR signals, was also present (Figure 2c). RpBphP1 mutations such as D607E become promising candidates for amino acid mutations, when combined with other mutations to improve the function of RpBphP1.

Furthermore, as a result of introducing a point mutation into RpBphP1, mutants, in which NIR signals of photoswitching proteins consisting of the present mutant and a wild-type RpPpsR2 become high, were found (Figure 3a and b). These mutations also become promising candidates for amino acid mutations, when combined with other mutations to improve the function of RpBphP1.

### 2-3. Functional analysis of photoswitching proteins consisting of wild-type RpBphP1 and RpPpsR2 mutant

Next, an RpPpsR2 mutant was prepared by introducing point mutations into RpPpsR2, and then, how to change the binding state of a wild-type RpBphP1 and the RpPpsR2 mutant under the dark and under near-infrared light irradiation was examined. As a result, mutants that reduce Dark leak signals compared to a wild-type RpPpsR2 (WT) were found (Figure 4a and b). Mutants that significantly reduced Dark leak signals could significantly increase gene activation by light irradiation. For example, an L110A mutant could increase the gene activation by light irradiation by 13.4 times (Figure 4a). On the other hand, such amino acid mutants as Q105T, which have the effect of reducing Dark leak signals without reducing NIR signals, was also present (Figure 4b). Mutations such as Q105T become promising candidates for amino acid mutations, when combined with other mutations to improve the function of RpPpsR2.

Furthermore, as a result of introducing a point mutation into RpPpsR2, mutants, in which NIR signals of photoswitching proteins consisting of the present mutant and a wild-type RpBphP1 become high, were found (Figure 5a to c). In particular, NIR signals of photoswitching proteins consisting of an RpPpsR2 mutant and a wild-type RpBphP1, which have a Q105S mutation, become high without being affected by Dark leak signals (Figure 5b). Such a mutation as Q105S becomes a promising candidate for amino acid mutation, when combined with other mutations to improve the function of RpPpsR2.

### 2-4. Functional analysis of photoswitching proteins consisting of wild-type RpBphP1 and RpPpsR2 domain-modified body

An approach that can reduce Dark leak signals without introducing point mutations into amino acids was attempted. The three-dimensional structure of a wild-type RpPpsR2 consists of 5 domains. The present inventors have found that the binding ability of the RpPpsR2 with RpBphP1 is changed, when some of these 5 domains are extracted (removed), or when the order of linking these domains to one another is changed, or when the number of domains is changed (domain shuffling) (Figure 6). For example, in photoswitching proteins consisting of an RpPpsR2 domain-modified body on which the domain shuffling D4-D1-D2 has been performed and a wild-type RpBphP1, the Dark leak signals were significantly reduced, and as a result, the light-dependent activation was increased to 7.2 times (Figure 6b). In addition, the Dark leak signals were reduced in the D3-D1-D2 domain-modified body without reducing the NIR signals (Figure 6b). Domain shuffling such as D3-D1-D2 is promising for improving the function of RpPpsR2 by combining it with other mutations.

Even in the domain shuffling, mutants that increase NIR signals were found (Figure 7). These domain shufflings may also become promising candidates for mutations that can be combined with other mutations to improve the function of RpPpsR2.

The present inventors found that the binding ability with RpBphP1 was also changed by deleting the N-terminal side or C-terminal side in the RpPpsR2 domain (domain-deleted body). For example, the N-terminal side or C-terminal side of one (D1-D2) of the domain shufflings of RpPpsR2 was deleted (see Figure 8a). In the domain-modified body, the binding ability with RpBphP1 is changed. For example, the domain-modified body D1-D2 (del C 15) significantly reduced the Dark leak signals and increased the light-dependent activation to 9.6 times (Figure 8b). Meanwhile, the domain-modified body D1-D2 (del N 77) reduced the Dark leak signals without reducing the NIR signals (Figure 8b). Such domain modifications comprising amino acid deletions are promising for the improvement of the function of RpPpsR2 by combination with other mutations.

Furthermore, even among domain-modified bodies comprising deletions, mutants with increased NIR signals were found (Figures 9a and b). In particular, D1-D2 (del N 64) increases NIR signals without changing Dark leak signals (Figure 9b). Such domain-modified bodies are promising for the improvement of the function of RpPpsR2 by combination with other mutations.

Next, with regard to D1-D2 (del N 93) (Figure 8b), which is one of the domain-modified bodies comprising deletions, amino acid-deleted bodies in the vicinity, ranging from (del N 88) to (del N 97), were examined for every single amino acid (see Figure 10a). As a result, it was found that all of the deleted bodies in the vicinity reduce the Dark leak signals (Figure 10b). The domain-modified bodies comprising such deletion are promising for the improvement of the function of RpPpsR2 by combination with other mutations.

Further, with regard to D1-D2 (del N 93), one of the domain-modified bodies comprising deletions, deleted bodies obtained by deleting the C-terminal side thereof were examined (see Figure 11a). As a result, it was found that the Dark leak signals are further reduced by deleting the C-terminal side (Figure 11b). The domain-modified bodies comprising such deletion are promising for the improvement of the function of RpPpsR2 by combination with other mutations.

### 2-5. Functional analysis of photoswitching proteins consisting of an RpBphP1 mutant and/or an RpPpsR2 mutant having multiple beneficial mutations (point mutations or domain modifications, etc.)

There was performed the functional analysis of photoswitching proteins consisting of an RpBphP1 mutant and/or an RpPpsR2 mutant, which have multiple beneficial mutations, among the amino acid point mutants or domain-modified bodies (domain-shuffled bodies and domain-deleted bodies) that have been functionally analyzed so far. Figure 12 shows possible combinations obtained when there are multiple mutations/modifications in RpBphP1 and RpPpsR2 that reduce Dark leak signals and multiple mutations/modifications that increase NIR signals. There are a total of 12 combination patterns. As a result of these combinations, it was found that photoswitching proteins having multiple beneficial mutations have functions that are further improved due to the cumulative effects of the mutations. A specific explanation is given below.

There were examined how to change the properties of photoswitching proteins when a point mutation or domain modification that reduces Dark leak signals and a point mutation or domain modification that increases NIR signals were simultaneously introduced into RpBphP1 or RpPpsR2. First, an example, in which the combination pattern of RpBphP1 and RpPpsR2 was Combination 2 in Figure 12, was examined. When a point mutation that reduces Dark leak signals and a point mutation that increases NIR signals were simultaneously introduced into RpBphP1, the Dark leak signals could be reduced with little reduction in the NIR signals of the photoswitching proteins (Figure 13a). Next, an example, in which the combination pattern of RpBphP1 and RpPpsR2 was Combination 7 in Figure 12, was examined. When a point mutation that reduces Dark leak signals and a point mutation that increases NIR signals were simultaneously introduced into RpPpsR2, the Dark leak signals could be reduced with little reduction in the NIR signals of the photoswitching proteins (Figure 13b). Furthermore, a mutant, in which the combination pattern of RpBphP1 and RpPpsR2 was Combination 7 in Figure 12 but both a domain modification and a point mutation were introduced into RpPpsR2, was examined. That is to say, an RpPpsR2 mutant having both a domain modification (D3-D2) that reduces Dark leak signals and a point mutation (L137E) that increases NIR signals was prepared, and this RpPpsR2 mutant was combined with a wild-type RpBphP1. As a result, it was possible to improve the ratio of the NIR signal intensity of the photoswitching proteins with respect to the Dark leak signal intensity of the photoswitching proteins (Figure 14).

As described above, it became clear that the function of the photoswitching proteins can be improved by introducing multiple mutations exhibiting beneficial effects on RpBphP1 or RpPpsR2 (point mutations or domain modifications, etc.) into the RpBphP1 or the RpPpsR2.

Next, there were examined how to change the properties of photoswitching proteins when beneficial mutations were introduced into both RpBphP1 and RpPpsR2 (Figure 15, Figure 16, Figure 17, and Figure 18). First, an example, in which the combination pattern of RpBphP1 and RpPpsR2 was Combination 9 in Figure 12, was examined. An RpBphP1 mutant with a point mutation that reduces Dark leak signals introduced was combined with an RpPpsR2 mutant with a domain shuffling modification that reduces Dark leak signals. As a result, the Dark leak signals could be reduced with little reduction in the NIR signals of the photoswitching proteins (Figure 15a). Next, an example, in which the combination pattern of RpBphP1 and RpPpsR2 was Combination 3 in Figure 12, was examined. An RpBphP1 mutant with a point mutation that reduces Dark leak signals introduced was combined within RpPpsR2 mutant with a point mutation that increases NIR signals introduced. As a result, the Dark leak signals could be reduced with little reduction in the NIR signals of the photoswitching proteins (Figure 15b).

Next, an example, in which the combination pattern of RpBphP1 and RpPpsR2 was Combination 4 in Figure 12, was examined. That is, an RpBphP1 mutant with a point mutation that reduces Dark leak signals and a point mutation that increases NIR signals introduced was combined with an RpPpsR2 mutant with a point mutation that increases NIR signals introduced. As a result, the Dark leak signals of the switching proteins could be reduced with little reduction in the NIR signals thereof (Figure 16a). Next, an example, in which the combination pattern of RpBphP1 and RpPpsR2 was Combination 10 in Figure 12, was examined. That is, an RpBphP1 mutant with a point mutation that reduces Dark leak signals and a point mutation that increases NIR signals introduced was combined with an RpPpsR2 mutant with a point mutation that reduces Dark leak signals introduced. As a result, even in this case, it was possible to improve the ratio of the NIR signal intensity of the photoswitching proteins with respect to the Dark leak signal intensity of the photoswitching proteins (Figure 16b).

Moreover, a case, in which the combination pattern of RpBphP1 and RpPpsR2 was Combination 3 in Figure 12 but RpPpsR2 had multiple point mutations that increase NIR signals, was examined. That is, an RpPpsR2 mutant with two point mutations that increase NIR signals introduced was combined with an RpBphP1 mutant with one point mutation that reduces Dark leak signals introduced. As a result, the Dark leak signals could be reduced with little reduction in the NIR signals of the photoswitching proteins (Figure 17a). Next, a case in which the combination pattern of RpBphP1 and RpPpsR2 was Combination 11 in Figure 12, namely, the combination of RpPpsR2 with one point mutation reducing Dark leak signals and one point mutation increasing NIR signals introduced and RpBphP1 with one point mutation reducing Dark leak signals introduced, was examined. As a result, even in this case, it was possible to improve the ratio of the NIR signal intensity of the photoswitching proteins with respect to the Dark leak signal intensity of the photoswitching proteins (Figure 17b).

Furthermore, a case, in which the combination pattern of RpBphP1 and RpPpsR2 was Combination 4 in Figure 12 but RpPpsR2 had multiple point mutations that increase NIR signals, was examined. That is, an RpPpsR2 mutant with two point mutations that increase NIR signals introduced was combined with an RpBphP1 mutant with one point mutation that reduces Dark leak signals and one point mutation that increases NIR signals introduced. As a result, even in this case, it was possible to improve the ratio of the NIR signal intensity of the photoswitching proteins with respect to the Dark leak signal intensity of the photoswitching proteins (Figure 18a). Moreover, a case, in which the combination pattern of RpBphP1 and RpPpsR2 was Combination 12 in Figure 12, was examined. That is, an RpBphP1 mutant with a point mutation that reduces Dark leak signals and a point mutation that increases NIR signals introduced was combined with an RpPpsR2 mutant with a point mutation that reduces Dark leak signals and a point mutation that increases NIR signals introduced. As a result, even in this case, it was possible to improve the ratio of the NIR signal intensity of the photoswitching proteins with respect to the Dark leak signal intensity of the photoswitching proteins (Figure 18b). The combination shown in Figure 18a and the combination shown in Figure 18b were able to cause light-dependent activation of more than 10 times (i.e., the ratio of the NIR signal intensity with respect to the Dark signal intensity became 10 or more). The aforementioned results show that the functions of the photoswitching proteins can be improved by introducing multiple mutations having beneficial effects into RpBphP1 and RpPpsR2.

Next, the combination pattern of RpBphP1 and RpPpsR2 was Combination 11 in Figure 12, but an RpPpsR2 mutant having both a domain modification and a point mutation was combined with an RpBphP1 mutant reducing Dark leak signals. As a result, even in this case, it was possible to improve the ratio of the NIR signal intensity of the photoswitching proteins with respect to the Dark leak signal intensity of the photoswitching proteins (Figure 19).

Furthermore, the combination pattern of RpBphP1 and RpPpsR2 was Combination 12 in Figure 12, but an RpPpsR2 mutant having both a domain modification and a point mutation was combined with an RpBphP1 mutant having multiple point mutations. That is, the RpPpsR2 mutant was combined with an RpBphP1 mutant having one point mutation reducing Dark leak signals and one point mutation increasing NIR signals introduced. As a result, even in this case, it was possible to improve the ratio of the NIR signal intensity of the photoswitching proteins with respect to the Dark leak signal intensity of the photoswitching proteins (Figure 20).

The aforementioned results show that the functions of the photoswitching proteins can be improved by combining all of the point mutations having beneficial effects of RpBphP1 and the domain modifications and point mutations of RpPpsR2.

### 2-6. Confirmation of gene activation by the photoswitching proteins of the present invention in cells and in vivo

### 2-6-1. Confirmation of genome gene activation in cells

Utilizing the near-infrared light manipulation tool (the aforementioned CRISPR-Cas9 system-based gene expression light manipulation tool (CPTS)), the functions of the photoswitching proteins of the present invention in cultured cells were analyzed.

Guide RNA for the light manipulation tool was redesigned to target each of the human genes (ASCL1, HBG1, MYOD1 or IL1R2). These four types of genes were simultaneously transfected into a human cell line, and near-infrared light irradiation was then performed. As a result, all of the four types of human genes could be simultaneously photoactivated (Figure 21a). How long it takes for this near-infrared light manipulation tool to activate genes after initiation of near-infrared light irradiation was examined. Light irradiation was initiated on the human cell line into which the light manipulation tool had been introduced, and the degree of activation of the light manipulation tool was quantitatively evaluated at the designated time. As a result, it was found that gene activation could be already induced 18 times at 1.5 hours after the light irradiation (Figure 21b). Further, whether the gene activation by this light manipulation tool was terminated when the light irradiation was stopped was examined. As a result, it was found that the gene activation could be terminated by stopping the light irradiation, and that the gene activation could be resumed by restarting the light irradiation (Figure 21c).

### 2-6-2. Confirmation of gene activation in vivo

Utilizing CPTS, the functions of the photoswitching proteins of the present invention in living mouse bodies was analyzed.

CPTS components were gene-transfected into mouse liver, and the mice were then irradiated with a light, using an LED panel, from outside the living mouse bodies (760 nm, 300 W/m²).

When CPTS for a luminescent reporter was used, no luminescence signals were observed in mice retained in the dark (Dark), but strong luminescence signals were observed in the liver of mice (NIR) irradiated with a near-infrared light (Figure 22b). Next, when CPTS for a mouse ASCL1 gene was used, the liver was excised from the mouse after near-infrared light irradiation (Figure 22c, left view), and then, mRNA was extracted and quantitatively analyzed. As a result, it could be confirmed that the ASCL1 gene was activated 47.8 times in the liver of mice (NIR) irradiated with a near-infrared light, compared to mice retained in the dark (Dark) (Figure 22c, right view).

### Industrial Applicability

The present invention provides photoswitching proteins whose binding and dissociation can be precisely controlled by turning light irradiation with a near-infrared light ON/OFF. Since the functions of the photoswitching proteins according to the present invention, which have been into a body, can be controlled from outside the body with a near-infrared light, the photoswitching proteins can be used for the purpose of treating diseases, etc., and thus, it is expected that they will be utilized in the medical field, etc.

## Claims

1. A protein set consisting of two proteins that bind to each other under near-infrared light irradiation, the protein set being a set of an RpBphP1 mutant and a wild-type RpPpsR2, a set of a wild-type RpBphP1 and an RpPpsR2 mutant, or a set of an RpBphP1 mutant and an RpPpsR2 mutant, wherein the binding of the protein set is weaker under the dark and/or is stronger under near-infrared light irradiation, than the binding of a wild-type RpBphP1 and a wild-type RpPpsR2.

2. The protein set according to claim 1, wherein the wild-type RpBphP1 is a protein consisting of the amino acid sequence as set forth in SEQ ID No: 1 or SEQ ID No: 2, or a protein having a sequence identity of 90% or more to the amino acid sequence as set forth in SEQ ID No: 1 or SEQ ID No: 2, and the wild-type RpPpsR2 is a protein consisting of the amino acid sequence as set forth in SEQ ID No: 3, or a protein having a sequence identity of 90% or more to the amino acid sequence as set forth in SEQ ID No: 3.

3. The protein set according to claim 2, which is a set of a wild-type RpPpsR2 and an RpBphP1 mutant, wherein
the RpBphP1 mutant is any of mutants consisting of amino acid sequences having one or multiple point mutations selected from the group of point mutations consisting of Q514A, D607E, R638A, H661A, R696A, E327A, E332A, E337A, D339A, E365A, Q367A, E429A, R490A, D498A, Q502A, Q502G, Q502V, Q502L, Q502I, Q502S, Q502T, Q502E, Q502K, Q502R, Q502H, Q502N, Q502F, Q502Y, Q502W, Q502C, Q502M, Q502P, F503A, R504A, R507A, E513G, E513D, E513P, E516A, F518A, S525A, D607A, D607G, D607V, D607L, D607I, D607S, D607T, D607K, D607R, D607H, D607N, D607Q, D607F, D607W, D607C, D607M, D607P, E613A, R636A, F639A, D654A, E678A, Y681A, E693A, H712D, H712E, H712P, R512A, E513V, E513A, E513L, E513T, E513M, and Q521A, in the amino acid sequence as set forth in SEQ ID No: 1 or SEQ ID No: 2.

4. The protein set according to claim 2, which is a set of a wild-type RpBphP1 and an RpPpsR2 mutant, wherein
the RpPpsR2 mutant is any of mutants consisting of amino acid sequences having one or multiple point mutations selected from the group of point mutations consisting of Q105T, T133A, L137A, N103A, Q105A, Q105C, Q105H, V107A, T108A, L110A, R113A, L114A, I115A, Q119A, M121A, M121E, R123A, D124A, Y125A, Y125E, W126A, R127A, R129A, E132A, R134A, R136A, Q105V, Q105S, K102A, Q105P, Q105R, L137E, V138A, V138E, F139E, and V146A, in the amino acid sequence as set forth in SEQ ID No: 3.

5. The protein set according to claim 2, which is a set of a wild-type RpBphP1 and an RpPpsR2 mutant, wherein
the RpPpsR2 mutant is a mutant consisting of an amino acid sequence, in which the amino acid sequence as set forth in SEQ ID No: 3 is modified to any of D1-D1-D2, D3-D1-D2, D1-D2 (del N 77), D1-D2, D2-D1, D2-D2, D3-D2, D3-D2 (L137E), D4-D2, D1-D2-D1, D1-D2-D2, D1-D2-D4, D2-D1-D2, D2-D3-D4, D4-D1-D2, D2-D3-D4-D1, D2-D3-D4-D2, D2-D3-D4-D3, D2-D3-D4-D5, D1-D2 (del N 88), D1-D2 (del N 89), D1-D2 (del N 90), D1-D2 (del N 91), D1-D2 (del N 92), D1-D2 (del N 93), D1-D2 (del N 94), D1-D2 (del N 95), D1-D2 (del N 96), D1-D2 (del N 97), D1-D2 (del C 5), D1-D2 (del C 15), D1-D2 (del N 93) (del C 5), D1-D2 (del N 93) (del C 6), D1-D2 (del N 93) (del C 11), D1-D2 (del N 93) (del C 12), D1-D2-D3 (del C 25), D1-D2-D3 (del C 41), D1-D2-D3 (del C 49), D1-D2-D3 (del C 70), D1-D2-D3 (del C 87), D1-D2-D3 (del C 96), D1-D2-D3 (del C 104), D1-D2 (del N 64), D2-D3, D1-D2-D3, D2-D2-D3, D2-D3-D1, D2-D3-D3, D3-D2-D3, D4-D2-D3, D1-D1-D2-D3, D1-D2-D3-D1, D1-D2-D3-D2, D1-D2-D3-D3, D1-D2-D3-D4, D2-D1-D2-D3, D2-D2-D3-D4, D2-D3-D4-D4, D3-D1-D2-D3, D3-D2-D3-D4, D1-D2 (del N 15), D1-D2 (del N 33), D1-D2 (del N 47), and D1-D2-D3 (del C 9).

6. The protein set according to claim 2, which is a set of an RpBphP1 mutant and an RpPpsR2 mutant, wherein
the RpBphP1 mutant is any of mutants consisting of amino acid sequences having one or multiple point mutations selected from the group of point mutations consisting of Q514A, D607E, R638A, H661A, R696A, E327A, E332A, E337A, D339A, E365A, Q367A, E429A, R490A, D498A, Q502A, Q502G, Q502V, Q502L, Q502I, Q502S, Q502T, Q502E, Q502K, Q502R, Q502H, Q502N, Q502F, Q502Y, Q502W, Q502C, Q502M, Q502P, F503A, R504A, R507A, E513G, E513D, E513P, E516A, F518A, S525A, D607A, D607G, D607V, D607L, D607I, D607S, D607T, D607K, D607R, D607H, D607N, D607Q, D607F, D607W, D607C, D607M, D607P, E613A, R636A, F639A, D654A, E678A, Y681A, E693A, H712D, H712E, H712P, R512A, E513V, E513A, E513L, E513T, E513M, and Q521A, in the amino acid sequence as set forth in SEQ ID No: 1 or SEQ ID No: 2, and
the RpPpsR2 mutant is any of mutants consisting of amino acid sequences having one or multiple point mutations selected from the group of point mutations consisting of Q105T, T133A, L137A, N103A, Q105A, Q105C, Q105H, V107A, T108A, L110A, R113A, L114A, I115A, Q119A, M121A, M121E, R123A, D124A, Y125A, Y125E, W126A, R127A, R129A, E132A, R134A, R136A, Q105V, Q105S, K102A, Q105P, Q105R, L137E, V138A, V138E, F139E, and V146A, in the amino acid sequence as set forth in SEQ ID No: 3.

7. The protein set according to claim 2, which is a set of an RpBphP1 mutant and an RpPpsR2 mutant, wherein
the RpBphP1 mutant is any of mutants consisting of amino acid sequences having one or multiple point mutations selected from the group of point mutations consisting of Q514A, D607E, R638A, H661A, R696A, E327A, E332A, E337A, D339A, E365A, Q367A, E429A, R490A, D498A, Q502A, Q502G, Q502V, Q502L, Q502I, Q502S, Q502T, Q502E, Q502K, Q502R, Q502H, Q502N, Q502F, Q502Y, Q502W, Q502C, Q502M, Q502P, F503A, R504A, R507A, E513G, E513D, E513P, E516A, F518A, S525A, D607A, D607G, D607V, D607L, D607I, D607S, D607T, D607K, D607R, D607H, D607N, D607Q, D607F, D607W, D607C, D607M, D607P, E613A, R636A, F639A, D654A, E678A, Y681A, E693A, H712D, H712E, H712P, R512A, E513V, E513A, E513L, E513T, E513M, and Q521A, in the amino acid sequence as set forth in SEQ ID No: 1 or SEQ ID No: 2, and
the RpPpsR2 mutant is a mutant consisting of an amino acid sequence, in which the amino acid sequence as set forth in SEQ ID No: 3 is modified to any of D1-D1-D2, D3-D1-D2, D1-D2 (del N 77), D1-D2, D2-D1, D2-D2, D3-D2, D3-D2 (L137E), D4-D2, D1-D2-D1, D1-D2-D2, D1-D2-D4, D2-D1-D2, D2-D3-D4, D4-D1-D2, D2-D3-D4-D1, D2-D3-D4-D2, D2-D3-D4-D3, D2-D3-D4-D5, D1-D2 (del N 88), D1-D2 (del N 89), D1-D2 (del N 90), D1-D2 (del N 91), D1-D2 (del N 92), D1-D2 (del N 93), D1-D2 (del N 94), D1-D2 (del N 95), D1-D2 (del N 96), D1-D2 (del N 97), D1-D2 (del C 5), D1-D2 (del C 15), D1-D2 (del N 93) (del C 5), D1-D2 (del N 93) (del C 6), D1-D2 (del N 93) (del C 11), D1-D2 (del N 93) (del C 12), D1-D2-D3 (del C 25), D1-D2-D3 (del C 41), D1-D2-D3 (del C 49), D1-D2-D3 (del C 70), D1-D2-D3 (del C 87), D1-D2-D3 (del C 96), D1-D2-D3 (del C 104), D1-D2 (del N 64), D2-D3, D1-D2-D3, D2-D2-D3, D2-D3-D1, D2-D3-D3, D3-D2-D3, D4-D2-D3, D1-D1-D2-D3, D1-D2-D3-D1, D1-D2-D3-D2, D1-D2-D3-D3, D1-D2-D3-D4, D2-D1-D2-D3, D2-D2-D3-D4, D2-D3-D4-D4, D3-D1-D2-D3, D3-D2-D3-D4, D1-D2 (del N 15), D1-D2 (del N 33), D1-D2 (del N 47), and D1-D2-D3 (del C 9).

8. An RpBphP1 mutant consisting of an amino acid sequence having any one or multiple point mutations selected from the following point mutations in the amino acid sequence as set forth in SEQ ID No: 1 or SEQ ID No: 2: Q514A, D607E, R638A, H661A, R696A, E327A, E332A, E337A, D339A, E365A, Q367A, E429A, R490A, D498A, Q502A, Q502G, Q502V, Q502L, Q502I, Q502S, Q502T, Q502E, Q502K, Q502R, Q502H, Q502N, Q502F, Q502Y, Q502W, Q502C, Q502M, Q502P, F503A, R504A, R507A, E513G, E513D, E513P, E516A, F518A, S525A, D607A, D607G, D607V, D607L, D607I, D607S, D607T, D607K, D607R, D607H, D607N, D607Q, D607F, D607W, D607C, D607M, D607P, E613A, R636A, F639A, D654A, E678A, Y681A, E693A, H712D, H712E, H712P, R512A, E513V, E513A, E513L, E513T, E513M, and Q521A.

9. An RpPpsR2 mutant consisting of an amino acid sequence having any one or multiple point mutations selected from the following point mutations in the amino acid sequence as set forth in SEQ ID No: 3: Q105T, T133A, L137A, N103A, Q105A, Q105C, Q105H, V107A, T108A, L110A, R113A, L114A, I115A, Q119A, M121A, M121E, R123A, D124A, Y125A, Y125E, W126A, R127A, R129A, E132A, R134A, R136A, Q105V, Q105S, K102A, Q105P, Q105R, L137E, V138A, V138E, F139E, and V146A.

10. An RpPpsR2 mutant consisting of an amino acid sequence, in which the amino acid sequence as set forth in SEQ ID No: 3 is modified to any of the following:
D1-D1-D2, D3-D1-D2, D1-D2 (del N 77), D1-D2, D2-D1, D2-D2, D3-D2, D3-D2 (L137E), D4-D2, D1-D2-D1, D1-D2-D2, D1-D2-D4, D2-D1-D2, D2-D3-D4, D4-D1-D2, D2-D3-D4-D1, D2-D3-D4-D2, D2-D3-D4-D3, D2-D3-D4-D5, D1-D2 (del N 88), D1-D2 (del N 89), D1-D2 (del N 90), D1-D2 (del N 91), D1-D2 (del N 92), D1-D2 (del N 93), D1-D2 (del N 94), D1-D2 (del N 95), D1-D2 (del N 96), D1-D2 (del N 97), D1-D2 (del C 5), D1-D2 (del C 15), D1-D2 (del N 93) (del C 5), D1-D2 (del N 93) (del C 6), D1-D2 (del N 93) (del C 11), D1-D2 (del N 93) (del C 12), D1-D2-D3 (del C 25), D1-D2-D3 (del C 41), D1-D2-D3 (del C 49), D1-D2-D3 (del C 70), D1-D2-D3 (del C 87), D1-D2-D3 (del C 96), D1-D2-D3 (del C 104), D1-D2 (del N 64), D2-D3, D1-D2-D3, D2-D2-D3, D2-D3-D1, D2-D3-D3, D3-D2-D3, D4-D2-D3, D1-D1-D2-D3, D1-D2-D3-D1, D1-D2-D3-D2, D1-D2-D3-D3, D1-D2-D3-D4, D2-D1-D2-D3, D2-D2-D3-D4, D2-D3-D4-D4, D3-D1-D2-D3, D3-D2-D3-D4, D1-D2 (del N 15), D1-D2 (del N 33), D1-D2 (del N 47), and D1-D2-D3 (del C 9).

11. A nucleic acid encoding any of the RpBphP1 mutants according to claim 8.

12. A nucleic acid encoding any of the RpPpsR2 mutants according to claim 9.

13. A nucleic acid encoding any of the RpPpsR2 mutants according to claim 10.
